# EUROPEAN PATENT APPLICATION

(11) **EP 2 669 281 A1**
(43) Date of publication of application: **04.12.2013**
(21) Application number: 12739658.8
(22) Date of filing: 27.01.2012
(51) Int. Cl.: C07D 401/12, C07D 211/44

(54) **METHOD FOR PRODUCING DI(ARYLAMINO)ARYL COMPOUND, AND SYNTHETIC INTERMEDIATE THEREFOR**

(30) Priority: 28.01.2011 JP 2011016110
(71) Applicant: Astellas Pharma Inc., Tokyo 103-8411 (JP)
(72) Inventor: OBITSU, Kazuyoshi, Tokyo 103-8411 (JP); AKIBA, Takahiro, Tokyo 103-8411 (JP); KOBAYASHI, Koji, Tokyo 103-8411 (JP); HIRASAWA, Shun, Tokyo 103-8411 (JP); KONDOH, Yutaka, Tokyo 103-8411 (JP); TAKEGUCHI, Kazuhiro, Tokyo 103-8411 (JP); TAKAHAMA, Yuji, Tokyo 103-8411 (JP); ORII, Ryoki, Tokyo 103-8411 (JP)
(74) Representative: HOFFMANN EITLE
(86) International application number: PCT/JP2012/051876
(87) International publication number: WO 2012/102393

(57) **Abstract**

Provided is a method for producing a di(arylamino)aryl compound that has superior inhibitory activity against the kinase activities of EML4-ALK fusion protein and mutant EGFR protein and is useful as an active ingredient in pharmaceutical compositions for cancer treatment. The production method includes no purification step using silica gel column chromatography or no step possibly producing a mutagenic mesylate ester as a by-product, greatly improves overall yield, and is high yield and low cost and suitable for the industrial production of pharmaceutical products. Also provided is a synthetic intermediate that is useful in the production method.

## Description

### TECHNICAL FIELD

The present invention relates to a method for producing N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (hereinafter, referred to as "compound of Formula (1)" in some cases), and to a synthetic intermediate therefor.

### BACKGROUND ART

We have already reported that the compound of Formula (1) has high inhibitory activity against EML4-ALK fusion protein kinase and mutant EGFR protein kinase and is useful as an active ingredient in pharmaceutical compositions for cancer treatment (Patent Document 1). Further, we have found and reported that there are five polymorphs of the compound of Formula (1) as A01-type, A02-type, A03-type, A04-type and A05-type and that A04-type crystal is the most stable among those crystals (Patent Document 2). 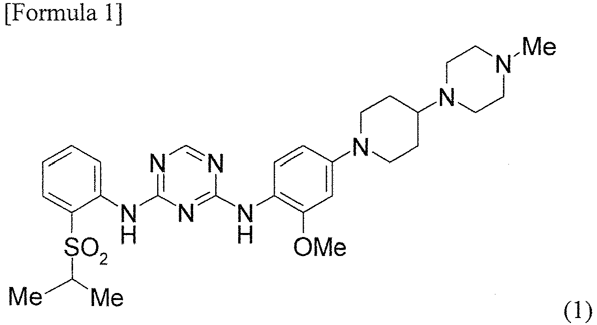 The method for producing a compound of Formula (1) described in Patent Document 1 (Example 23 of Patent Document 1) is shown by Reaction Scheme (I), according to the Preparation Examples and Examples described in the same document. More specifically, it is a method for producing the intended compound of Formula (1) in which 2,4-dichloro-1,3,5-triazine (hereinafter, "compound of Formula (15)" in some cases) and 2-(isopropylsulfonyl)aniline (hereinafter, "compound of Formula (8)" in some cases) are reacted in accordance with the method described in Preparation Example 7 of the same document to give the compound of Formula (14) (Preparation Example 22 of Patent Document 1), and subsequently, the obtained compound of Formula (14) and 2-methoxy-4-[4-(4-methylpiperazin-1-yl)piperidin-1-yl]aniline (hereinafter, "compound of Formula (13)" in some cases) produced by a known method (for example, refer to International Publication No. WO 2005/016894 pamphlet) are reacted in accordance with the method described in Example 1 of the same document.

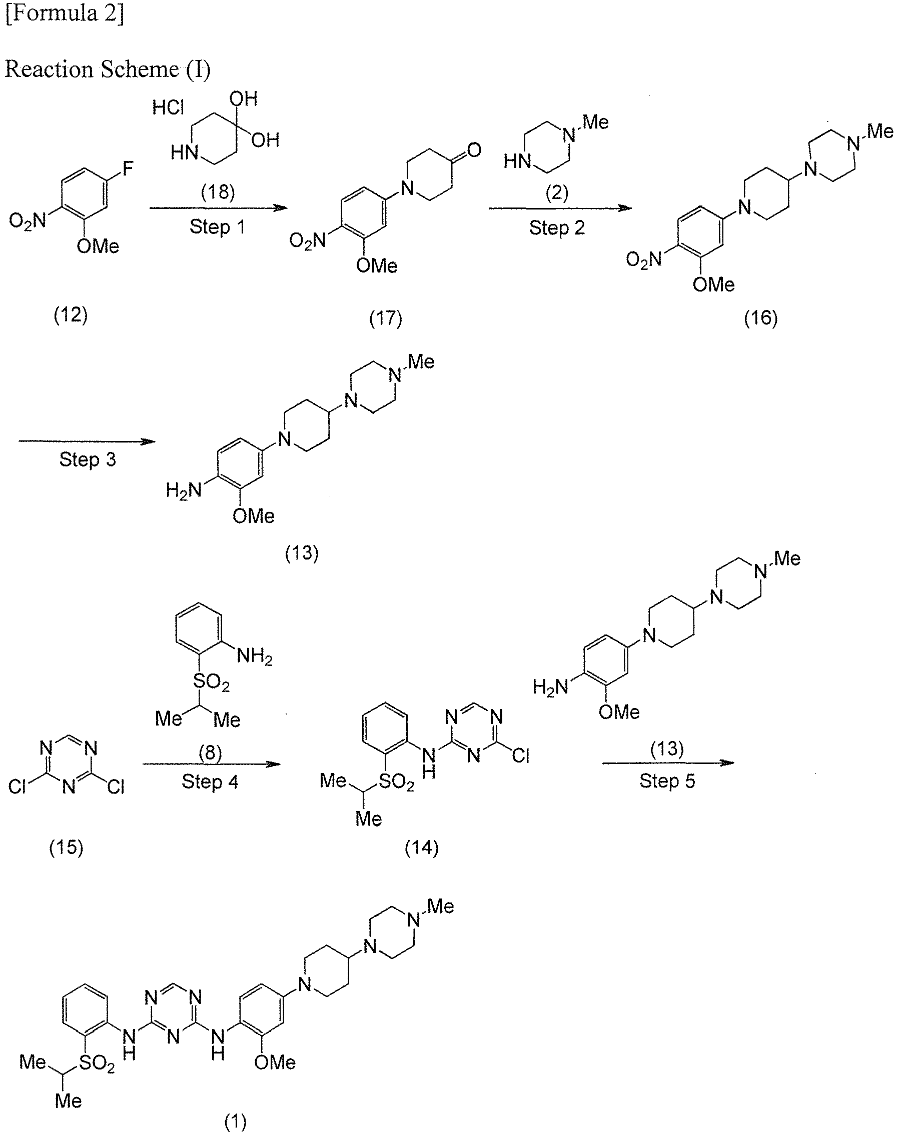

However, as indicated in, for example, Table 3 shown later, the method for producing the compound of Formula (1) disclosed in Patent Document 1 includes steps with low product yields such as about 50% yields, and the overall yield of the compound of Formula (1), which is the intended final product, is only on the order of 10%. Since the method has yield and cost problems, it is in no way satisfactory in industrial production. Further, the production method shown in Reaction Scheme (I) is desired to be improved for industrial production of pharmaceutical products for the following reasons: it has to be admitted that the compound of Formula (15) is unsuitable as a starting material in industrial production since the compound has problems about stable procurement (the scale on which the compound is available, the period of time required for production) and cost (the price of the compound); and the method includes steps that require purification using silica gel column chromatography (Steps 2, 4 and 5 in Reaction Scheme (I)) and a step that possibly produces a mutagenic mesylate ester as a by-product (Step 5 in Reaction Scheme (I)).

### CITATION LIST

### PATENT DOCUMENTS

Patent Document 1: International Publication No. WO 2009/008371 pamphlet
Patent Document 2: International Publication No. WO 2011/145548 pamphlet

### SUMMARY OF INVENTION

### TECHNICAL PROBLEM

An object of the present invention is to provide a method for producing the compound of Formula (1) which is high yield and low cost and suitable for the industrial production of pharmaceutical products and provide a synthetic intermediate that is useful in the production method.

### SOLUTION TO PROBLEM

As a result of intensive and extensive studies on industrial methods for producing the compound of Formula (1), the present inventors have found that the use of predetermined starting materials and synthetic intermediates enables the compound of Formula (1) to be produced by a method that includes neither a step requiring purification using silica gel column chromatography nor a step possibly producing a mutagenic mesylate ester as a by-product and which greatly improves overall yield and is high yield and low cost and suitable for the industrial production of pharmaceutical products. This finding led to the completion of the present invention. In summary, the present invention relates to a method for producing the compound of Formula (1) shown below and to a synthetic intermediate therefor.

[1] A method for producing the compound of Formula (1): 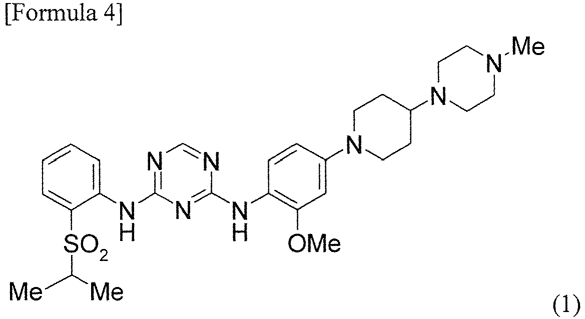 comprising subjecting the compound of Formula (3): 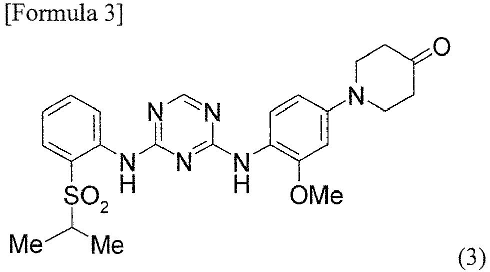 to a reductive amination reaction by allowing 1-methylpiperazine (the compound of Formula (2) in Reaction Scheme (II) shown later) to act on the compound of Formula (3).
[2] The method according to [1], wherein the compound of Formula (3) is produced by a method comprising subjecting a compound of Formula (4): 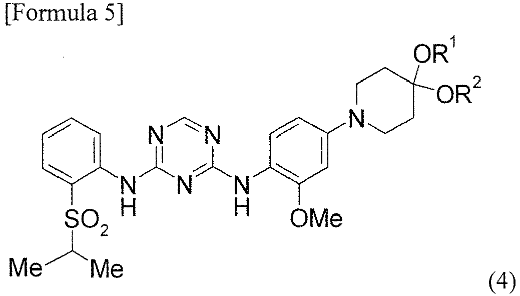 (wherein R¹ and R², which may be the same or different, are lower alkyl, or R¹ and R² taken together may form lower alkylene)
   to a deketalization reaction.
[3] The method according to [2], wherein the compound of Formula (4) is produced by a method comprising subjecting a compound of Formula (5): 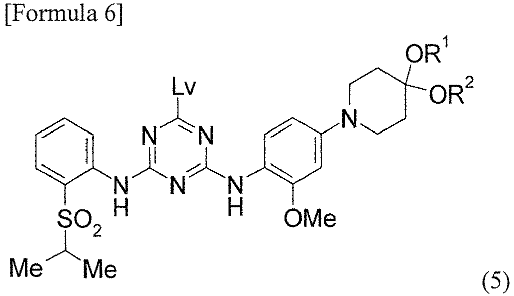 (wherein R¹ and R², which may be the same or different, are lower alkyl, or R¹ and R² taken together may form lower alkylene; Lv is a leaving group)
   to a hydrogenation reaction.
[4] The method according to [3], wherein the compound of Formula (5) is produced by a method comprising subjecting a compound of Formula (7): 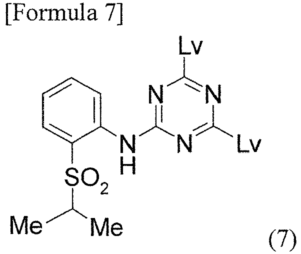 (wherein Lv, which may be the same or different, are leaving groups)
   to an aromatic nucleophilic substitution reaction by allowing a compound of Formula (6): 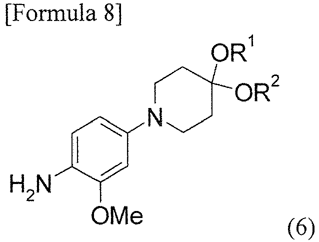 (wherein R¹ and R², which may be the same or different, are lower alkyl, or R¹ and R² taken together may form lower alkylene)
   to act on the compound of Formula (7).
[5] The method according to [4], wherein the compound of Formula (7) is produced by a method comprising subjecting a compound of Formula (9): 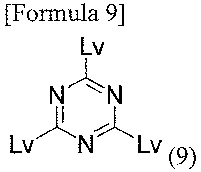 (wherein Lv, which may be the same or different, are leaving groups)
   to an aromatic nucleophilic substitution reaction by allowing 2-(isopropylsulfonyl)aniline (the compound of Formula (8) in Reaction Scheme (II) shown later) to act on the compound of Formula (9).
[6] The method according to [4] or [5], wherein the compound of Formula (6) is produced by a method comprising:
   subjecting a compound of Formula (12): 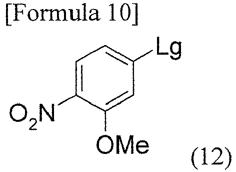 (wherein Lg is a leaving group)
      to an aromatic nucleophilic substitution reaction by allowing a compound of Formula (11): 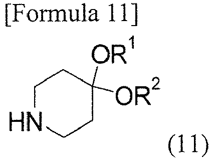 (wherein R¹ and R², which may be the same or different, are lower alkyl, or R¹ and R² taken together may form lower alkylene)
      to act on the compound of Formula (12); and
      then performing a hydrogenation reaction.
[7] A compound represented by Formula (4') or a salt thereof: 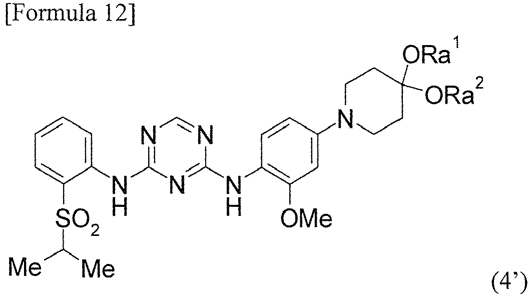 (wherein Ra¹ and Ra², which may be the same or different, are lower alkyl, or Ra¹ and Ra² taken together may form lower alkylene, provided that Ra¹ and Ra² taken together do not form dimethylene).
[8] A compound represented by Formula (5) or a salt thereof: 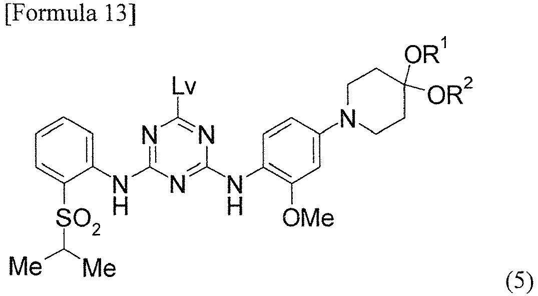 (wherein R¹ and R², which may be the same or different, are lower alkyl, or R¹ and R² taken together may form lower alkylene; Lv is a leaving group).
[9] The compound according to [8] or a salt thereof, wherein Lv is a leaving group selected from the group consisting of halogen, sulfonyloxy groups, lower alkylsulfanyl and lower alkylsulfonyl.
[10] The compound according to [9], wherein Lv is halogen.
[11] The compound according to [10], wherein Lv is Cl and R¹ and R² are both methyl groups.
[12] The compound according to [7] or a salt thereof, wherein Ra¹ and Ra² are both methyl groups.
[13] A compound represented by Formula (6') or a salt thereof: 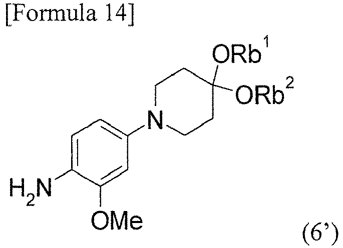 (wherein Rb¹ and Rb², which may be the same or different, are lower alkyl, or Rb¹ and Rb² taken together may form lower alkylene, provided that Rb¹ and Rb² taken together do not form dimethylene).
[14] The compound according to [13], wherein Rb¹ and Rb² are both methyl groups.
[15] The method according to [5], wherein the compound of Formula (9) is cyanuric chloride.
[16] The method according to any of [1] to [6], further comprising a purification step including crystallization of the compound of Formula (1).

It is to be noted that the compounds disclosed in the present invention may be in free form or may form salts. Specific examples of forms of the salts include acid addition salts with inorganic acids (e.g., hydrochloric acid, hydrobromic acid, hydroiodic acid, sulfuric acid, nitric acid, phosphoric acid) or with organic acids (e.g., formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, glutamic acid), salts with inorganic bases (e.g., sodium, potassium, magnesium, calcium, aluminum) or with organic bases (e.g., methylamine, ethylamine, ethanolamine, lysine, ornithine), salts with various amino acids and amino acid derivatives (e.g., acetylleucine), ammonium salts, and the like.

Further, the compounds disclosed in the present invention may be free forms or salts thereof in the form of various hydrates, solvates or crystalline polymorphs. The present invention further encompasses methods for producing synthetic intermediates using the free forms or salts. The compounds disclosed in the present invention may also be labeled with various radioactive or non-radioactive isotopes, and the present invention further encompasses methods for producing synthetic intermediates using the labeled compounds.

As described above, the compound of Formula (1) may be in free form or may form salts, or may be various hydrates or solvates of the free forms or salts, or may be crystalline or amorphous forms of the hydrates or solvates. In one embodiment, the compound of Formula (1) is a crystal. In one embodiment, the compound of Formula (1) is a crystal of the free form. Another embodiment is A01-type, A02-type, A03-type, A04-type, A05-type or A06-type crystal of the free-form compound of Formula (1). Yet another embodiment is A04-type crystal of the free-form compound of Formula (1). Yet another embodiment is A06-type crystal of the free-form compound of Formula (1).

The A06-type crystal as referred to herein is a novel crystal of the compound of Formula (1) which is obtained by the methods described in the Examples of the present invention. The physicochemical properties of the A06-type crystal are described below.
[1] It has heat absorption peak around 125°C in a DSC analysis (rate of temperature increase: 30°C/min).
[2] It has peaks around 2θ (°) = 6.7, 9.0, 9.8, 13.0, 13.7, 14.3, 15.2, 15.6, 16.3, 17.1, 18.1, 18.4, 19.3, 19.8, 20.1, 20.4, 20.9, 22.3, 23.0, 23.7, 24.7, 25.6, 26.1, 27.1, 28.2, 29.3, 29.8, 30.5, 32.3 and 34.2 in a powder X-ray diffraction. Distinctive peaks include peaks around 2θ (°) = 6.7, 9.0, 9.8, 13.7, 16.3, 19.3, 20.1 and 20.9.

The measurement conditions for the above analyses are as described below.
TA Instruments Q-2000 was used in the measurement for the DSC analysis according to the following conditions: temperature range of measurement: room temperature to 220°C; rate of temperature increase: 30°C/min; nitrogen flow rate: 50 mL/min; aluminum sample pan.
Miniflex was used in the measurement of the powder X-ray diffraction according to the following conditions: X-ray tube: Cu; tube-current: 15 mA; tube-voltage: 30 kV; sampling width: 0.010°; scanning speed: 2.0°/min; range of measurement diffraction angles (2θ): 3-35°.

It is to be noted that the spacing of the crystal lattice and the overall pattern are important in identifying crystals in light of the nature of the powder X-ray diffraction data, and the relative intensity should not be strictly interpreted since it can change slightly according to the crystal growth direction, the particle size and the measurement conditions.
Further, the term "around", which takes in account various errors during the measurement, indicates ±3°C for one embodiment and ±2°C for another embodiment in the DSC analysis and ±2°for one embodiment and ±1°for another embodiment in the powder X-ray diffraction.

Furthermore, some of the compounds disclosed in the present invention may have tautomers or geometrical isomers, depending on the type of substituents. Even when a compound having an isomer appears herein only in one isomer form, the present invention encompasses the other isomers, and also encompasses separated isomers or mixtures thereof.

### ADVANTAGEOUS EFFECTS OF INVENTION

The present invention can provide a method for producing the compound of Formula (1) which is high yield and low cost and suitable for the industrial production of pharmaceutical products, and can provide a synthetic intermediate that is useful in the production method.

### BRIEF DESCRIPTION OF DRAWINGS

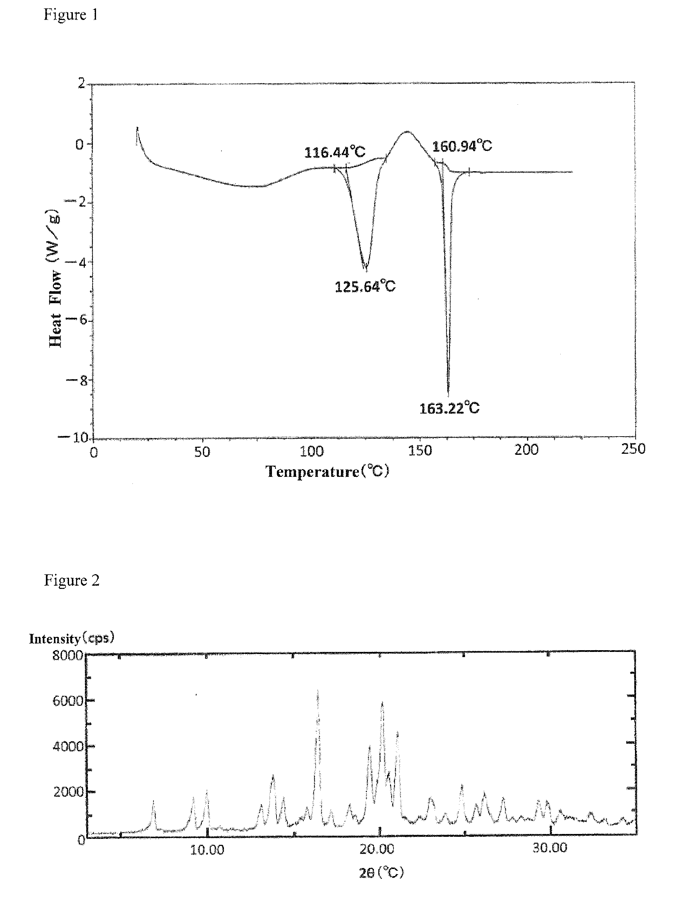

Figure 1 shows a DSC chart of A06-type crystal.
Figure 2 shows a powder X-ray diffraction pattern of A06-type crystal.

### DESCRIPTION OF EMBODIMENTS

The present invention is described in detail below.
As used herein, the term "halogen" refers to F, Cl, Br, or I. Another embodiment is F, and yet another embodiment is Cl.

The term "lower alkyl" refers to linear or branched alkyl having 1 to 6 carbon atoms including methyl, ethyl, n-propyl, isopropyl, n-butyl, isobutyl, sec-butyl, tert-butyl, n-pentyl, n-hexyl, and the like. Another embodiment is linear or branched alkyl having 1 to 4 carbon atoms, and yet another embodiment is methyl, ethyl, n-propyl, or isopropyl. Yet another embodiment is methyl.

The term "lower alkylene" refers to linear or branched alkylene having 1 to 6 carbon atoms including methylene, ethylene (or dimethylene), trimethylene, tetramethylene, pentamethylene, hexamethylene, propylene, methylmethylene, ethylethylene, 1,2-dimethylethylene, 1,1,2,2-tetramethylethylene, and the like. Another embodiment is linear or branched alkylene having 1 to 4 carbon atoms, and yet another embodiment is dimethylene or trimethylene. Yet another embodiment is dimethylene.

A method for producing the compound of Formula (1) of the present invention (Steps 1 to 7) is shown by Reaction Scheme (II), and embodiments of each of Steps 1 to 7 in that order are concretely described below.

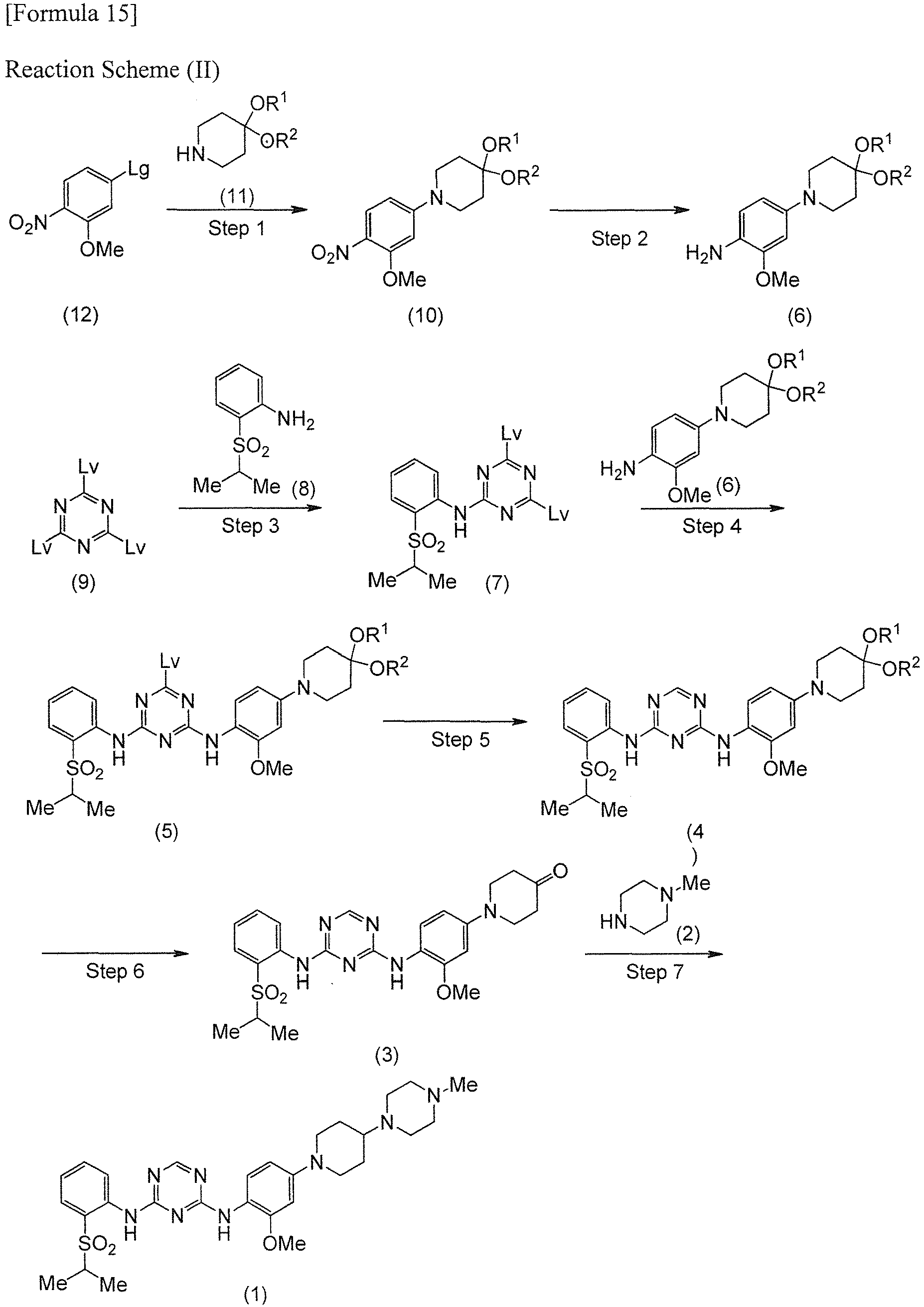

(In Reaction Scheme (II), Lg and Lv are leaving groups, and R¹ and R², which may be the same or different, are lower alkyl, or R¹ and R² taken together may form lower alkylene. The same applies hereinafter.)

### Step 1

This step is intended to produce a compound of Formula (10) by subjecting a compound of Formula (12) which has a leaving group Lg to an aromatic nucleophilic substitution reaction by allowing a compound of Formula (11) which is a piperidine derivative to act on the compound of Formula (12). Examples of the leaving group Lg as referred to herein include halogen, sulfonyloxy groups such as methanesulfonyloxy, p-toluenesulfonyloxy and trifluoromethanesulfonyloxy, lower alkylsulfanyl such as methanesulfanyl, ethanesulfanyl and n-propanesulfanyl, and lower alkylsulfonyl such as methanesulfonyl, ethanesulfonyl and n-propanesulfonyl. One embodiment is halogen. Another embodiment is F or Cl, and yet another embodiment is F.
In the reaction of this step, the compound of Formula (12) and the compound of Formula (11) are used in equal amounts or one of them is used in an excessive amount. In one embodiment, about 1 equivalent, for example, 0.95 to 1.20 equivalents of the compound of Formula (11) is used relative to the compound of Formula (12). A mixture of these compounds is stirred in a solvent inert to the reaction or in the absence of a solvent, generally for 0.1 hour to 5 days under cooling to heating to reflux (at 0°C to 80°C for one embodiment and 0°C to 40°C for another embodiment). Examples of the solvent used for this purpose include, but are not particularly limited to, aromatic hydrocarbons (e.g., benzene, toluene, xylene), ethers (e.g., diethyl ether, tetrahydrofuran, dioxane, dimethoxyethane), halogenated hydrocarbons (e.g., dichloromethane, 1,2-dichloroethane, chloroform), alcohols (e.g., methanol, ethanol, 2-propanol), ketones (e.g., acetone, 2-butanone), esters (e.g., ethyl acetate, isopropyl acetate), N,N-dimethylformamide, N,N-dimethylacetamide, N-methyl-2-pyrrolidone, 1,3-dimethyl-2-imidazolidinone, dimethyl sulfoxide, acetonitrile, and mixtures thereof. One embodiment is N,N-dimethylformamide. The reaction may be performed in the presence of an organic base (e.g., triethylamine, N,N-diisopropylethylamine, N-methylmorpholine, lithium diisopropylamide, n-butyl lithium, 1,8-diazabicyclo[5.4.0]undec-7-ene or 1,5-diazabicyclo[4.3.0]non-5-ene) or an inorganic base (e.g., potassium carbonate, sodium carbonate, sodium hydrogen carbonate, sodium hydroxide, potassium hydroxide or sodium hydride) (for one embodiment, 1,8-diazabicyclo[5.4.0]undec-7-ene), because it is advantageous for smooth reaction in some cases.

### [Documents]

S. R. Sandler and W. Karo, "Organic Functional Group Preparations", second edition, vol. 1, Academic Press Inc., 1991
The Chemical Society of Japan (ed.), "The Fifth Series of Experimental Chemistry", vol. 14 (2005) (Maruzen Co., Ltd.)

### Step 2

This step is intended to produce a compound of Formula (6) which has an amino group by subjecting the compound of Formula (10) which has a nitro group to a hydrogenation reaction to reduce the compound of Formula (10).
In the reaction of this step, the compound of Formula (10) is stirred in a solvent inert to the reaction in the presence of a metal catalyst, generally for 1 hour to 5 days under a hydrogen atmosphere at normal pressure to 50 atmospheres. This reaction is generally performed under cooling to heating (at 0°C to 40°C for one embodiment). Examples of the solvent used for this purpose include, but are not particularly limited to, alcohols (e.g., methanol, ethanol, 2-propanol), ethers (e.g., diethyl ether, tetrahydrofuran, dioxane, dimethoxyethane), water, ethyl acetate, N,N-dimethylformamide, dimethyl sulfoxide and mixtures thereof. One embodiment is tetrahydrofuran. The metal catalyst suitably used is a palladium catalyst (e.g., palladium carbon, palladium black, palladium hydroxide), a platinum catalyst (e.g., platinum plate, platinum oxide), a nickel catalyst (e.g., reduced nickel, Raney nickel), a cobalt catalyst (e.g., Raney cobalt), a rhodium catalyst (e.g., chlorotristriphenylphosphine rhodium), an iron catalyst (e.g., reduced iron) or the like. In one embodiment, palladium carbon or a platinum catalyst such as platinum plate or platinum oxide is used. In another embodiment, palladium carbon is used. Instead of hydrogen gas, formic acid, ammonium formate, potassium formate or the like can be also used as a hydrogen source in an amount equal to that of the compound of Formula (10) or in an excessive amount.

### [Documents]

M. Hudlicky, "Reductions in Organic Chemistry, 2nd ed. (ACS Monograph: 188), ACS, 1996
The Chemical Society of Japan (ed.), "The Fifth Series of Experimental Chemistry", vol. 19 (2005) (Maruzen Co., Ltd.)

### Step 3

This step is intended to produce a compound of Formula (7) by subjecting a compound of Formula (9) which has leaving groups Lv to an aromatic nucleophilic substitution reaction by allowing the compound of Formula (8) which is an aniline derivative to act on the compound of Formula (9). Examples of the leaving groups Lv as referred to herein, which may be the same or different, include halogen, sulfonyloxy groups such as methanesulfonyloxy·, p-toluenesulfonyloxy and trifluoromethanesulfonyloxy, lower alkylsulfanyl, and lower alkylsulfonyl. One embodiment is halogen. Another embodiment is F or Cl, and yet another embodiment is Cl for all the groups Lv.
In the reaction of this step, the compound of Formula (9) and the compound of Formula (8) are used in equal amounts or one of them is used in an excessive amount. In one embodiment, about 1 equivalent, for example, 0.95 to 1.2 equivalents of the compound of Formula (8) is used relative to the compound of Formula (9). A mixture of these compounds is stirred in a solvent inert to the reaction or in the absence of a solvent, generally for 0.1 hour to 5 days under cooling to heating to reflux (at 0°C to 80°C for one embodiment and 0°C to 40°C for another embodiment). Examples of the solvent used for this purpose include, but are not particularly limited to, aromatic hydrocarbons (e.g., benzene, toluene, xylene), ethers (e.g., diethyl ether, tetrahydrofuran, dioxane, dimethoxyethane), halogenated hydrocarbons (e.g., dichloromethane, 1,2-dichloroethane, chloroform), alcohols (e.g., methanol, ethanol, 2-propanol), ketones (e.g., acetone, 2-butanone), esters (e.g., ethyl acetate, isopropyl acetate), N,N-dimethylformamide, N,N-dimethylacetamide, N-methyl-2-pyrrolidone, 1,3-dimethyl-2-imidazolidinone, dimethyl sulfoxide, acetonitrile and mixtures thereof. One embodiment is acetone. The reaction may be performed in the presence of an organic base (e.g., triethylamine, N,N-diisopropylethylamine, N-methylmorpholine, lithium diisopropylamide, n-butyl lithium, 1,8-diazabicyclo[5.4.0]undec-7-ene or 1,5-diazabicyclo[4.3.0]non-5-ene) or an inorganic base (e.g., potassium carbonate, sodium carbonate, sodium hydrogen carbonate, sodium hydroxide, potassium hydroxide or sodium hydride) (for one embodiment, sodium hydrogen carbonate), because it is advantageous for smooth reaction in some cases.
When the reaction is performed in the presence of such a base as shown above, the starting compounds can be decomposed, or the intended reaction can not proceed or can only proceed poorly in some cases, depending on the properties or the like of the starting compounds. In these cases, the reaction may be performed in the presence of a mineral acid (e.g., hydrochloric acid, hydrobromic acid), an organic acid (e.g., acetic acid, propionic acid, trifluoroacetic acid) or a sulfonic acid (e.g., methanesulfonic acid, p-toluenesulfonic acid), because it is advantageous for smooth reaction in some cases.

### Step 4

This step is intended to produce a compound of Formula (5) by subjecting the compound of Formula (7) which has leaving groups Lv to an aromatic nucleophilic substitution reaction by allowing the compound of Formula (6) which is an aniline derivative to act on the compound of Formula (7). Examples of the leaving groups Lv as referred to herein, which may be the same or different, include halogen, sulfonyloxy groups such as methanesulfonyloxy, p-toluenesulfonyloxy and trifluoromethanesulfonyloxy, lower alkylsulfanyl, and lower alkylsulfonyl. One embodiment is halogen. Another embodiment is F or Cl, and yet another embodiment is Cl for the both groups Lv.
In the reaction of this step, the compound of Formula (7) and the compound of Formula (6) are used in equal amounts or one of them is used in an excessive amount. In one embodiment, about 1 equivalent, for example, 0.95 to 1.20 equivalents of the compound of Formula (6) is used relative to the compound of Formula (7). A mixture of these compounds is stirred in a solvent inert to the reaction or in the absence of a solvent, generally for 0.1 hour to 5 days under cooling to heating to reflux (at 0°C to 80°C for one embodiment and 0°C to 40°C for another embodiment). Examples of the solvent used for this purpose include, but are not particularly limited to, aromatic hydrocarbons (e.g., benzene, toluene, xylene), ethers (e.g., diethyl ether, tetrahydrofuran, dioxane, dimethoxyethane), halogenated hydrocarbons (e.g., dichloromethane, 1,2-dichloroethane, chloroform), alcohols (e.g., methanol, ethanol, 2-propanol), ketones (e.g., acetone, 2-butanone), esters (e.g., ethyl acetate, isopropyl acetate), N,N-dimethylformamide, N,N-dimethylacetamide, N-methyl-2-pyrrolidone, 1,3-dimethyl-2-imidazolidinone, dimethyl sulfoxide, acetonitrile and mixtures thereof. One embodiment is tetrahydrofuran. The reaction may be performed in the presence of an organic base (e.g., triethylamine, N,N-diisopropylethylamine, N-methylmorpholine, lithium diisopropylamide, n-butyl lithium, 1,8-diazabicyclo[5.4.0]undec-7-ene or 1,5-diazabicyclo[4.3.0]non-5-ene) or an inorganic base (e.g., potassium carbonate, sodium carbonate, sodium hydrogen carbonate, sodium hydroxide, potassium hydroxide or sodium hydride) (for one embodiment, N,N-diisopropylethylamine), because it is advantageous for smooth reaction in some cases.
It is to be noted that this step and Steps 5 and 6 which are described later may be collectively performed as successive reaction steps, because it is advantageous in terms of yield in some cases.

### Step 5

This step is intended to produce a compound of Formula (4) by subjecting the compound of Formula (5) which has a leaving group Lv to a hydrogenation reaction to reduce the compound of Formula (5) for removal of the leaving group Lv. Examples of the leaving group Lv as referred to herein include halogen, sulfonyloxy groups such as methanesulfonyloxy, p-toluenesulfonyloxy and trifluoromethanesulfonyloxy, lower alkylsulfanyl, and lower alkylsulfonyl. One embodiment is halogen. Another embodiment is F or Cl, and yet another embodiment is Cl.
In the reaction of this step, the compound of Formula (5) is stirred in a solvent inert to the reaction in the presence of a metal catalyst, generally for 1 hour to 5 days under a hydrogen atmosphere at normal pressure to 50 atmospheres. This reaction is generally performed under cooling to heating (at 0°C to 40°C for one embodiment). Examples of the solvent used for this purpose include, but are not particularly limited to, alcohols (e.g., methanol, ethanol, 2-propanol), ethers (e.g., diethyl ether, tetrahydrofuran, dioxane, dimethoxyethane), water, ethyl acetate, N,N-dimethylformamide, dimethyl sulfoxide and mixtures thereof. One embodiment is tetrahydrofuran. The metal catalyst suitably used is a palladium catalyst (e.g., palladium carbon, palladium black, palladium hydroxide), a platinum catalyst (e.g., platinum plate, platinum oxide), a nickel catalyst (e.g., reduced nickel, Raney nickel), a cobalt catalyst (e.g., Raney cobalt), a rhodium catalyst (e.g., chlorotristriphenylphosphine rhodium), an iron catalyst (e.g., reduced iron) or the like. In one embodiment, palladium carbon or a platinum catalyst such as platinum plate or platinum oxide is used. In another embodiment, palladium carbon is used. Instead of hydrogen gas, formic acid, ammonium formate, potassium formate or the like can be also used as a hydrogen source in an amount equal to that of the compound of Formula (5) or in an excessive amount.
The reaction may be performed in the presence of an organic base (e.g., triethylamine, N,N-diisopropylethylamine or N-methylmorpholine) or an inorganic base (e.g., potassium carbonate, sodium carbonate or potassium hydroxide), because it is advantageous for smooth reaction in some cases.
It is to be noted that this step and subsequent Step 6 may be collectively performed as successive reaction steps, because it is advantageous in terms of yield in some cases.

### Step 6

This step is intended to produce the compound of Formula (3) which has a ketone group by subjecting the compound of Formula (4) which has a ketal group to a deketalization reaction.
The reaction of this step can be performed by reference to "Protective Groups in Organic Synthesis" written by Greene and Wuts, third edition, John Wiley & Sons Inc., 1999. For example, the reaction is performed by subjecting the compound of Formula (4) to a hydrolysis reaction in a water-containing solvent at room temperature to under heating to reflux, under acidic conditions.
It is to be noted that the embodiments in which R¹ and R² in Reaction Scheme (II), which may be the same or different, are lower alkyl (for example, an embodiment in which they are both methyl) are advantageous for smooth reaction of this step in some cases.

### Step 7

This step is intended to produce the compound of Formula (1) by subjecting the compound of Formula (3) to a reductive amination reaction by allowing the compound of Formula (2) (1-methylpiperazine) which is a pyrazine derivative to act on the compound of Formula (3).
In the reaction of this step, the compound of Formula (3) and the compound of Formula (2) are used in equal amounts or one of them is used in an excessive amount. In one embodiment, about 2 equivalents, for example, 1.8 to 2.2 equivalents of the compound of Formula (2) is used relative to the compound of Formula (3). A mixture of these compounds is stirred in a solvent inert to the reaction, generally for 0.1 hour to 5 days at -45°C to under heating to reflux, preferably at 0°C to room temperature, in the presence of a reducing agent (for one embodiment, about 2 equivalents, for example, 1.8 to 2.2 equivalents of a reducing agent relative to the compound of Formula (3)). Examples of the solvent used for this purpose include, but are not particularly limited to, alcohols (e.g., methanol, ethanol), ethers (e.g., diethyl ether, tetrahydrofuran, dioxane, dimethoxyethane), aromatic hydrocarbons (e.g., benzene, toluene, xylene) and mixtures thereof. One embodiment is toluene. The reducing agent is, for example, sodium cyanoborohydride, sodium triacetoxyborohydride, or sodium borohydride. In one embodiment, sodium triacetoxyborohydride is used. It is preferable in some cases to perform the reaction in the presence of a dehydrating agent such as molecular sieves or an acid such as acetic acid, hydrochloric acid or a titanium(IV) isopropoxide complex (acetic acid for one embodiment and 3.6 to 4.4 equivalents of acetic acid for another embodiment). Alternatively, the compound of Formula (1) may be obtained by isolating imine produced through condensation between the compounds of Formulae (3) and (2) and reducing the imine intermediate.
Instead of a treatment with the reducing agent described above, a reduction catalyst also allows the reaction to be performed in the presence or absence of an acid such as acetic acid or hydrochloric acid. In this case, the reaction is performed under cooling to heating, under a hydrogen atmosphere at normal pressure to 50 atmospheres. The reduction catalyst suitably used is metal catalysts listed in Step 2 or 5 or other catalysts. In one embodiment, palladium carbon or a platinum catalyst such as platinum plate or platinum oxide is used. In another embodiment, palladium carbon is used. Examples of the solvent used for this purpose include, but are not particularly limited to, alcohols (e.g., 2-propanol), ethers (e.g., diethyl ether, tetrahydrofuran, dioxane, dimethoxyethane), water, ethyl acetate, N,N-dimethylformamide, dimethyl sulfoxide and mixtures thereof. One embodiment is tetrahydrofuran. Instead of hydrogen gas, formic acid, ammonium formate, potassium formate or the like can be also used as a hydrogen source.
It is to be noted that the use of the reduction catalyst described above in the reaction of this step is advantageous in terms of side-reaction suppression and yield in some cases.

### [Documents]

A. R. Katritzky and R. J. K. Taylor, "Comprehensive Organic Functional Group Transformations II", vol. 2, Elsevier Pergamon, 2005
The Chemical Society of Japan (ed.), "The Fifth Series of Experimental Chemistry", vol. 14 (2005) (Maruzen Co., Ltd.)

### Purification step including crystallization

This Step 7 may further include a purification step including crystallization of the compound of Formula (1) if desired. By including this step, an intended high-purity crystal of the compound of Formula (1) can be produced. The purification step including crystallization means a step for producing the compound of Formula (1) by crystallization from a solvent in which the compound is dissolved. One embodiment is a step including crystallization using a seed crystal of the compound of Formula (1). Another embodiment is a step including subjecting the solvent in which the compound of Formula (1) is dissolved to an activated carbon treatment before performing crystallization. Yet another embodiment is a step including crystallization of the compound of Formula (1) using a seed crystal to give A04-type crystal as the most stable crystal.
Methods for producing the compound of Formula (1) as A04-type crystal are described below. It is to be noted that the production conditions can be changed as appropriate depending on intended crystals.
(Method A) High-purity A04-type crystal, which is the most stable crystal, can be produced by subjecting to this process the compound of Formula (1), which is produced in Step 7.
   In this process, the compound of Formula (1) is stirred in a solvent inert to the reaction, generally for 0.5 to 24 hours under heating (at 50°C to 80°C for one embodiment and 65°C for another embodiment). After that, the solvent is concentrated, and a seed crystal is added thereto, followed by stirring generally for 0.5 to 24 hours under heating (at 60°C to 80°C for one embodiment and 70°C for another embodiment). Subsequently, the mixture obtained is cooled and further stirred for 1 to 24 hours, and the resulting crystal is collected by filtration. Examples of the solvent used for this purpose include, but are not particularly limited to, ketones such as acetone and 2-butanone.
(Method B) The compound of Formula (1) and an appropriate amount of activated carbon that is, for example, 1/50 to 1/2 of the amount of the compound of Formula (1) based on weight ratio (about 1/10 of the amount of the compound of Formula (1) for one embodiment), are stirred in a solvent inert to the reaction, generally for 1 to 24 hours under heating (at 50°C to 100°C for one embodiment and 75°C for another embodiment), followed by filtration of activated carbon. After the same treatment of activated carbon is performed once to several times (three times for one embodiment), the solvent is concentrated, a seed crystal is added thereto, and the resulting mixture is stirred generally for 1 to 24 hours under heating (at 60°C to 80°C for one embodiment and 70°C for another embodiment). Subsequently, the mixture is cooled and further stirred for 1 to 24 hours, and the resulting crystal is collected by filtration. Examples of the solvent used for this purpose include, but are not particularly limited to, the solvents used in Method A.

The each compound shown in Reaction Scheme (II) is isolated and purified as a free compound or as a salt (for example, a pharmaceutically acceptable salt), hydrate, solvate or crystalline polymorph thereof. A pharmaceutically acceptable salt of the each compound in Reaction Scheme (II) can also be produced by performing a conventional salt-forming reaction.
The isolation and purification are performed by employing common chemical operations such as extraction, fractional crystallization, and various types of fractionation chromatography.

It is to be noted that R¹ and R² in Reaction Scheme (II) for one embodiment, which may be the same or different, are lower alkyl, or R¹ and R² taken together may form lower alkylene (provided that R¹ and R² taken together do not form dimethylene). In another embodiment, R¹ and R², which may be the same or different, are lower alkyl. In yet another embodiment, R¹ and R² are both methyl.

One embodiment of the compound of Formula (4') is (i) a compound wherein Ra¹ and Ra², which may be the same or different, are lower alkyl. Another embodiment is (ii) the compound wherein Ra¹ and Ra² are both methyl.

One embodiment of the compound of Formula (5) is (i) a compound wherein R¹ and R², which may be the same or different, are lower alkyl. Another embodiment is (ii) a compound wherein R¹ and R² are both methyl. Yet another embodiment is (iii) a compound wherein Lv is a leaving group selected from the group consisting of halogen, sulfonyloxy groups, lower alkylsulfanyl and lower alkylsulfonyl. Yet another embodiment is (iv) a compound wherein Lv is halogen. Yet another embodiment is (v) a compound wherein Lv is Cl. Yet another embodiment is (vi) a compound that falls under (iii), (iv) or (v) above and (1) or (2) above. Yet another embodiment is (vii) the compound wherein Lv is Cl and R¹ and R² are both methyl.

One embodiment of the compound of Formula (6') is a compound wherein Rb¹ and Rb², which may be the same or different, are lower alkyl. Another embodiment is the compound wherein Rb¹ and Rb² are both methyl.

A known method for producing the compound of Formula (1) shown in Reaction Scheme (I) is specifically described below as a reference example. It is to be noted that, as used herein, Me represents a methyl group, D1 indicates δ (ppm) of ¹H-NMR peaks in chloroform-d, D2 indicates δ (ppm) of ¹H-NMR peaks in dimethylsulfoxide-d₆, "EI" indicates EI-MS[M]⁺, "ESI+" indicates ESI-MS[M+H]⁺, and "ESI-" indicates ESI-MS[M-H]⁻. It is also to be noted that when the term "343K" is used, it indicates a measurement result at 343K (70°C).

### (Reference Example)

### Step 1 Synthesis of 1-(3-methoxy-4 nitrophenyl)piperidin-4-one (the compound of Formula (17))

To a mixture of 4-fluoro-2-methoxy-1-nitrobenzene (4.00 g), potassium carbonate (8.00 g) and N,N-dimethylformamide (40 mL), 4-piperidone monohydrate hydrochloride (4.40 g) was added, followed by stirring overnight at 70°C. To the reaction mixture, water (150 mL) was added, and the resulting mixture was extracted twice with ethyl acetate (80 mL). After the extract was washed with water and saturated brine and dried over anhydrous sodium sulfate, the solvent was distilled off under reduced pressure. The resulting residue was washed with diethyl ether to give 1-(3-methoxy-4 nitrophenyl)piperidin-4-one (4.62 g, 78.9% yield) as an ocher solid.
D1: 2.58-2.70(4H,m),3.76-3.84(4H,m),3.98(3H,m),6.34(1H,d,J=2.5Hz),6.45(1H,dd,J=2.5,9.3Hz),8.04(1H,d,J=9.3Hz)
It is to be noted that the instrumental date shown above is the data of 1-(3-methoxy-4 nitrophenyl)piperidin-4-one obtained by the same reaction as in this step.

### Step 2 Synthesis of 1-[1-(3-methoxy-4-nitrophenyl)piperidin-4 yl]-4-methylpiperazine (the compound of Formula (16))

To a mixture of 1-(3-methoxy-4 nitrophenyl)piperidin-4-one (4.62 g) and 1,2-dichloroethane (60 mL), N-methylpiperazine (2.50 mL) was added, and the resulting mixture was stirred for 30 minutes. After that, sodium triacetoxyborohydride (4.78 g) was added thereto, followed by stirring overnight at room temperature. To the reaction mixture, a saturated aqueous sodium hydrogen carbonate solution (150 mL) was added and the resulting mixture was extracted twice with chloroform (50 mL). After the extract was dried over anhydrous sodium sulfate, the solvent was distilled off under reduced pressure. The resulting residue was purified by silica gel column chromatography (eluent; chloroform:methanol:saturated aqueous ammonia = 30:1:0.1 to 15:1:0.1) and then washed with hexane to give 1-[1-(3-methoxy-4-nitrophenyl)piperidin-4 yl]-4-methylpiperazine (4.52 g, 73.2% yield) as a yellow solid.

D1: 1.55-1.70(2H,m),1.87-2.04(2H,m),2.31(3H,s),2.38-2.77(9H,m),2.89-3.05(2H,m),3.83-4.06(5H,m),6.31(1H,d,J=2.6Hz),6.42(1H,dd,J=2.6,9.4Hz), 7.99(1H,d,J=9.4Hz)

### Step 3 Synthesis of 2-methoxy-4-[4-(4-methylpiperazin-1-yl)piperidin-1-yl]aniline (the compound of Formula (13))

To a mixture of 1-[1-(3-methoxy-4-nitrophenyl)piperidin-4 yl]-4-methylpiperazine (2.18 g) and ethanol (50 mL), 5% palladium carbon (600 mg) was added, followed by stirring at room temperature for 8 hours under a hydrogen atmosphere at normal pressure. After filtration through celite, the solvent was distilled off under reduced pressure to give 2-methoxy-4-[4-(4-methylpiperazin-1-yl)piperidin-1-yl]aniline (1.96 g, 98.8% yield) as a light-purple solid.
D1: 1.64-1.80(2H,m),1.87-1.99(2H,m),2.26-2.80(11H,m),3.36-3.63(4H,m),3.83(3H,s),6.42(1H,dd,J=2.3,8.3Hz),6.52(1H,d,J=2.3Hz),6.64(1H,d,J=8.3Hz) EI: 304

### Step 4 Synthesis of 4-chloro-N-[2-(propane-2-sulfonyl)phenyl]-1,3,5-triazin-2-amine (the compound of Formula (14))

To a mixture of 2,4-dichloro-1,3,5-triazine (the compound of Formula (15), 56.7 g) and tetrahydrofuran (500 mL), a mixture of 2-(propane-2-sulfonyl)aniline (the compound of Formula (8), 50 g), N,N-diisopropylethylamine (44.0 mL) and tetrahydrofuran (500 mL) was added dropwise, followed by stirring at room temperature for 14 hours. After that, the compound of Formula (15) (18.9 g) and N,N-diisopropylethylamine (22 mL) were added thereto, and the resulting mixture was further stirred at room temperature for 3 days. To the reaction mixture, water (300 mL) and a saturated aqueous sodium hydrogen carbonate solution (200 mL) were added, and the resulting mixture was extracted three times with ethyl acetate. An organic layer obtained was washed with saturated brine and dried over anhydrous magnesium sulfate. After the solvent was distilled off, ethyl acetate and diethyl ether were added to the resulting residue, and a precipitated solid as a by-product was removed by filtration. The resulting residue was purified by silica gel column chromatography (eluent; hexane:ethyl acetate = 1:0 to 1:1) and washed with diethyl ether to give 4-chloro-N-[2-(propane-2-sulfonyl)phenyl]-1,3,5-triazin-2-amine (43.1 g, 55.9% yield).
D2: 1.14(6H,d,J=6.8Hz),3.45-3.57(1H,m),7.52-7.60(1H,m),7.80-7.87(1H,m),7.89-7.97(2H,m),8.64(1H,s),10.17(1H,s)

### Step 4 (Alternative method) Synthesis of 4-chloro-N-[2-(propane-2-sulfonyl)phenyl]-1,3,5-triazin-2-amine (the compound of Formula (14))

To a mixture of 2,4-dichloro-1,3,5-triazine (the compound of Formula (15), 460 mg) and tetrahydrofuran (5 mL), a mixture of 2-(propane-2-sulfonyl)aniline (the compound of Formula (8), 600 mg), N,N-diisopropylethylamine (0.58 mL) and tetrahydrofuran (10 mL) was added dropwise, followed by stirring at room temperature for 3 days. To the reaction mixture, water (60 mL) and a saturated aqueous sodium hydrogen carbonate solution (20 mL) were added, and the resulting mixture was extracted twice with ethyl acetate (40 mL). An organic layer obtained was washed with saturated brine and dried over anhydrous sodium sulfate. The solvent was distilled off and the resulting residue was purified by silica gel column chromatography (eluent; chloroform) to give 4-chloro-N-[2-(propane-2-sulfonyl)phenyl]-1,3,5-triazin-2-amine (340 mg, 36.1% yield).
D1: 1.32(6H,d,J=6.8Hz),3.16-3.28(1H,m),7.30-7.36(1H,m),7.68-7.55(1H,m),7.90-7.95(1H,m),8.50(1H,d,J=8.4Hz),8.61(1H,s),9.88(1H,br)
ESI+: 313

### Step 5 Synthesis of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (the compound of Formula (1))

To a mixture of 2-methoxy-4-[4-(4-methylpiperazin-1-yl)piperidin-1-yl]aniline (the compound of Formula (13), 29.2 g) and ethanol (450 mL), methanesulfonic acid (19.0 mL) was added, and the resulting mixture was stirred at room temperature for 15 minutes. Subsequently, 4-chloro-N-[2-(propane-2-sulfonyl)phenyl]-1,3,5-triazin-2-amine (the compound of Formula (14), 30.0 g) was added thereto, followed by stirring at 100°C for 5 hours. After the reaction mixture was allowed to cool, diethyl ether (900 mL) was added thereto and a precipitated solid was collected by filtration. The obtained solid was dissolved in water (300 mL) and adjusted to pH8 with a saturated aqueous sodium hydrogen carbonate solution, and then the resulting mixture was extracted three times with ethyl acetate (300 mL). After an organic layer obtained was washed with water and saturated brine and then dried over anhydrous sodium sulfate, the solvent was distilled off under reduced pressure. The resulting residue was purified by silica gel column chromatography (eluent; chloroform:methanol:saturated aqueous ammonia = 100:1:0 to 20:1:0.1) and then washed with ethanol to give N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (27.9 g, 50.1% yield) as a pale beige solid.
D1: 1.31(6H,d,J=6.8Hz),1.59-1.78(2H,m),1.90-2.01(2H,m),2.24-2.80(14H,m),3.19-3.32(1H,m),3.65-3.75(2H,m),3.88(3H,s),6.50-6.59(2H,m),7.18-7.30(1H,m),7.53-7.70(2H,m),7.88(1H,dd,J=1.4,8.0Hz),8.10(1H,br),8.37(1H,br),8.53(1H,br),9.29(1H,s)

Next, the methods for producing the compound of Formula (1) that are shown by Reaction Scheme (II) according to the present invention are specifically described below by means of Examples 1 and 2. It should be noted that the present invention is not limited to these Examples, and the skilled person can make appropriate modification and alteration by a method obvious to the skilled person unless they are against the purpose of the present invention. Naturally, such modification and alteration are also included by the present invention. Further, the each compound as a starting material can be produced by a method obvious to the skilled person. It is to be noted that Example 2 is an example in which a large-scale (kilogram-scale) synthesis was performed by the same method as in Example 1. The purity of the compound of Formula (1) described in the Examples was calculated in HPLC area%, and the proportion of A04-type crystal was calculated by dividing the melting heat [J/g] of the A04-type crystal obtained in a DSC analysis by the sum of the same melting heat and the melting heat [J/g] of crystals mixed with the A04-type crystal.

### EXAMPLES

### Example 1

### Step 1 Synthesis of 4,4-dimethoxy-1-(3-methoxy-4-nitrophenyl)piperidine (the compound of Formula (10) wherein R¹ and R² are both methyl)

After 4,4-dimethoxypiperidine monohydrochloride (35.9 g) and N,N-dimethylformamide (75 mL) were mixed, 1,8-diazabicyclo[5.4.0]undec-7-ene (57.5 mL) was added to the mixture. To the resulting mixture, 5-fluoro-2-nitroanisole (30.0 g) prepared separately and N,N-dimethylformamide (30 mL) were added at room temperature, followed by stirring for 5 hours. To the reaction mixture, water (120 mL) was added at room temperature, and the resulting mixture was stirred for 4.5 hours. After that, a precipitated crystal was collected by filtration. The obtained crystal was washed with a mixture of N,N-dimethylformamide and water (1:1) (60 mL), water (60 mL), and additional water (60 mL) in that order and then dried at 40°C under reduced pressure to give 4,4-dimethoxy-1-(3-methoxy-4-nitrophenyl)piperidine (49.9 g, 96.1% yield) as a crystal.
D2: 1.72-1.80(4H,m),3.14(6H,s),3.44-3.50(4H,m),3.91(3H,m),6.52(1H,d,J=2.4Hz),6.60(1H,dd,J=2.4,9.2Hz),7.88(1H,d,J=9.2Hz)
ESI+: 297

### Step 2 Synthesis of 4-(4,4-dimethoxypiperidin-1-yl)-2-methoxyaniline (the compound of Formula (6) wherein R¹ and R² are both methyl)

After 4,4-dimethoxy-1-(3-methoxy-4-nitrophenyl)piperidine (45.0 g) and tetrahydrofuran (225 mL) were mixed, 5% palladium carbon (about 50% wet product, 4.5 g) was added to the mixure at room temperature, followed by stirring at room temperature for 5.5 hours under a hydrogen atmosphere (2.4821 × 10⁵ Pa). After that, palladium carbon was removed by filtration and washed with tetrahydrofuran (90 mL), and then the filtrate was concentrated under reduced pressure to make a total volume of about 90 mL to give a slurry. After the slurry was stirred at 40°C for 1 hour, n-heptane (135 mL) was added thereto, and the resulting mixture was stirred at 40°C for 1 hour and then cooled to 0°C. To the mixture, n-heptane (405 mL) was added and a precipitated crystal was collected by filtration. The obtained crystal was washed with a mixture of tetrahydrofuran (9 mL) and n-heptane (54 mL) and dried at 40°C under reduced pressure to give 4-(4,4-dimethoxypiperidin-1-yl)-2-methoxyaniline (37.9 g, 93.7% yield) as a crystal.
D2: 1.72-1.80(4H,m),2.90-2.97(4H,m),3.11(6H,s),3.73(3H,m),4.21(1H,br),6.30(1H,d,J=2.4,8.4Hz),6.46_6.56(2H,m) ESI+: 267

### Step 3 Synthesis of 4,6-dichloro-N-[2-(propane-2-sulfonyl)phenyl]-1,3,5-triazin-2-amine (the compound of Formula (7) wherein Lv are both Cl)

Cyanuric chloride (25.0 g), sodium hydrogen carbonate (13.7 g), 2-(isopropylsulfonyl)aniline (29.7 g) and acetone (200 mL) were mixed, followed by stirring at room temperature for 25 hours. To the reaction mixture, water (200 mL) was added at room temperature, and then the resulting mixture was stirred for 19 hours and a precipitated crystal was collected by filtration. The obtained crystal was washed with a mixture of acetone and water (1:1) (100 mL) and then dried at 40°C under reduced pressure to give 4,6-dichloro-N-[2-(propane-2-sulfonyl)phenyl]-1,3,5-triazin-2-amine (45.1 g, 95.8% yield) as a crystal.
D1: 1.32(6H,d,J=6.8Hz),3.22(1H,sept,J=6.8Hz),7.37(1H,m),7.74(1H,m),7.93(1H,m),8.44(1H,m), 10.02(1H,br)
ESI-: 345,347

### Step 4 Synthesis of 6-chloro-N-[4-(4,4-dimethoxypiperidin-1-yl)-2-methoxyphenyl]-N'-[2-(propane-2-sulfonyl)phenyl]-1,3,5-triazine-2,4-diamine (the compound of Formula (5) wherein Lv is Cl and R¹ and R² are both methyl)

After 4,6-dichloro-N-[2-(propane-2-sulfonyl)phenyl]-1,3,5-triazin-2-amine (40.0 g) and tetrahydrofuran (400 mL) were mixed, 4-(4,4-dimethoxypiperidin-1-yl)-2-methoxyaniline (32.2 g) and N,N-diisopropylethylamine (16.38 g) were added to the mixture at room temperature, and the resulting mixture was stirred for 4 hours. After that, isopropyl acetate (40 mL) and then a mixture of potassium carbonate (2.0 g) and water (40 mL) were added thereto for extraction. An organic layer obtained was concentrated under reduced pressure to make a total volume of about 200 mL and seeded with a crystal (4 mg) of 6-chloro-N-[4-(4,4-dimethoxypiperidin-1-yl)-2-methoxyphenyl]-N'-[2-(propane-2-sulfonyl)phenyl]-1,3,5-triazine-2,4-diamine as a seed crystal. The resulting mixture was stirred for about 15 minutes to give a slurry. To this slurry, n-heptane (200 mL) was added, and a mixture obtained was cooled to 0°C and stirred for 18 hours. A precipitated crystal was collected by filtration. The obtained crystal was washed with a mixture of tetrahydrofuran (40 mL) and n-heptane (40 mL) and then dried at 40°C under reduced pressure to give 6-chloro-N-[4-(4,4-dimethoxypiperidin-1-yl)-2-methoxyphenyl]-N'-[2-(propane-2-sulfonyl)phenyl]-1,3,5-triazine-2,4-diamine (61.4 g, 92.4% yield) as a crystal.
D1: 1.30(6H,d,J=6.8Hz),1.88-1.92(4H,m),3.18-3.26(1H,m),3.23(3H,s),3.87(1H,br),6.53(2H,br),7.21-7.23(1H,m),7.62(1H,br),7.88(1H,d,J=7.9Hz),8.05(1H,br),8.48(1H,br),9.41(1H,br)
ESI-: 575,577

Alternative method for Step 4 (an example using no seed crystal) Synthesis of 6-chloro-N-[4-(4,4-dimethoxypiperidin-1-yl)-2-methoxyphenyl]-N'-[2-(propane-2-sulfonyl)phenyl]-1,3,5-triazine-2,4-diamine (the compound of Formula (5) wherein Lv is Cl and R¹ and R² are both methyl)
After 4,6-dichloro-N-[2-(propane-2-sulfonyl)phenyl]-1,3,5-triazin-2-amine (23.0 g) and tetrahydrofuran (230 mL) were mixed, 4-(4,4-dimethoxypiperidin-1-yl)-2-methoxyaniline (18.5 g) and N,N-diisopropylethylamine (12.7 mL) were added to the mixture at room temperature, followed by stirring for 2 hours. After that, isopropyl acetate (57.5 mL) and then a mixture of potassium carbonate (5.75 g) and water (115 mL) were added thereto for extraction. An organic layer obtained was concentrated under reduced pressure. To the resulting residue, tetrahydrofuran (50 mL) was added and a mixture obtained was stirred to give a slurry. To this slurry, tetrahydrofuran (75 mL) and n-heptane (75 mL) were added, followed by stirring at 40°C for 1 hour. Subsequently, the resulting mixture was cooled to 0°C and further stirred for 18 hours. After that, n-heptane (50 mL) was added thereto and a precipitated crystal was collected by filtration. The obtained crystal was washed with a mixture of tetrahydrofuran and n-heptane (5:7) (24 mL) and then dried at 40°C under reduced pressure to give 6-chloro-N-[4-(4,4-dimethoxypiperidin-1-yl)-2-methoxyphenyl]-N'-[2-(propane-2-sulfonyl)phenyl]-1,3,5-triazine-2,4-diamine (30.6 g, 80.0% yield) as a crystal.

D1: 1.30(6H,d,J=6.8Hz),1.88-1.92(4H,m),3.18-3.26(1H,m),3.23(3H,s),3.87(1H,br),6.53(2H,br),7.21-7.23(1H,m),7.62(1H,br),7.88(1H,d,J=7.9Hz),8.05(1H,br),8.48(1H,br),9.41(1H,br)
ESI-: 575,577

### Steps 5 and 6 (successive steps) Synthesis of 1-[3-methoxy-4-({4-[2-(propane-2-sulfonyl)anilino]-1,3,5-triazin-2-yl}amino)phenyl]piperidin-4-one (the compound of Formula (3))

After 6-chloro-N-[4-(4,4-dimethoxypiperidin-1-yl)-2-methoxyphenyl]-N'-[2-(propane-2-sulfonyl)phenyl]-1,3,5-triazine-2,4-diamine (60.0 g), tetrahydrofuran (540 mL) and 10% palladium carbon (about 50% wet product, 10.7g) were mixed, N,N-diisopropylethylamine (16.11 g) and 2-propanol (60 mL) were added to the mixture. The resulting mixture was stirred at 40°C for 7 hours under a hydrogen atmosphere (2.4131×10⁵ Pa). Palladium carbon was removed by filtration and washed with tetrahydrofuran (120 mL). To the filtrate obtained, activated carbon (12.0 g) was added, followed by stirring overnight at room temperature. After that, activated carbon was removed by filtration and washed with tetrahydrofuran (120 mL) to give a solution comprising N-[4-(4,4-dimethoxypiperidin-1-yl)-2-methoxyphenyl]-N'-[2-(propane-2-sulfonyl)phenyl]-1,3,5-triazine-2,4-diamine. To this solution, a mixture of 35% hydrochloric acid (21.7 g) and water (120 mL) was added, and the resulting mixture was stirred at room temperature for 21.5 hours. To the reaction mixture, a mixture of potassium carbonate (35.9 g) and water (120 mL) was added for extraction. Activated carbon (12.0 g) was added to an organic layer obtained, and the resulting mixture was stirred for 16 hours and then filtered, and activated carbon was washed with tetrahydrofuran (120 mL). The filtrate obtained was concentrated under reduced pressure to make a total volume of about 120 mL. After acetone (180 mL) was added to the mixture obtained, the resulting mixture was seeded with a crystal (60 mg) of 1-[3-methoxy-4-({4-[2-(propane-2-sulfonyl)anilino]-1,3,5-triazin-2-yl}amino)phenyl]piperidin-4-one as a seed crystal. After stirring for 1 hour, water (480 mL) was added thereto, the resulting mixture was stirred for 20 hours, and a precipitated crystal was collected by filtration. The obtained crystal was washed with a mixture of acetone (36 mL) and water (96 mL) and dried at 40°C under reduced pressure to give 1-[3-methoxy-4-({4-[2-(propane-2-sulfonyl)anilino]-1,3,5-triazin-2-yl}amino)phenyl]piperidin-4-one (45.8 g, 88.7% yield (yield of the successive two steps)) as a crystal.
D2,343K: 1.17(6H,d,J=6.8Hz),2.46-2.50(4H,m),3.40(1H,sept,J=6.8Hz),3.61(4H,dd,J=6.1,6.2Hz),3.79(3H,s),6.57(1H,dd,J=2.6,8. 7Hz),6.70(1H,d,J=2.6Hz),7.25-7.29(1H,m),7.38(1H,d,J=8.7Hz),7.61(1H,br),7.77-7.80(1H,m),8.28(1H,s),8.50(1H,br),8.66(1H,br),9.25(1H,br)
ESI+: 497

### Step 5 Synthesis of N-[4-(4,4-dimethoxypiperidin-1-yl)-2-methoxyphenyl]-N'-[2-(propane-2-sulfonyl)phenyl]-1,3,5-triazine-2,4-diamine (the compound of Formula (4) wherein R¹ and R² are both methyl)

After 6-chloro-N-[4-(4,4-dimethoxypiperidin-1-yl)-2-methoxyphenyl]-N'-[2-(propane-2-sulfonyl)phenyl]-1,3,5-triazine-2,4-diamine (5.0 g), tetrahydrofuran (45 mL), 2-propanol (5 mL) and 10% palladium carbon (about 50% wet product, 1.0 g) were mixed, N,N-diisopropylethylamine (1.81 mL) was added to the mixture. The resulting mixture was stirred at 40°C for 5.5 hours under a hydrogen atmosphere (2.4821 ×10⁵ Pa). After palladium carbon was removed by filtration and washed with tetrahydrofuran (10 mL), 10% brine (20 mL) was added thereto for extraction. An organic layer obtained was concentrated under reduced pressure. To a concentrated residue, acetone (10 mL) and diisopropylether (40 mL) were added, and the resulting mixture was stirred for 30 minutes and a precipitated crystal was collected by filtration. The obtained crystal was washed with diisopropylether (20 mL) and dried at 40°C under reduced pressure to give N-[4-(4,4-dimethoxypiperidin-1-yl)-2-methoxyphenyl]-N'-[2-(propane-2-sulfonyl)phenyl]-1,3,5-triazine-2,4-diamine (4.31 g, 91.6% yield) as a crystal.
D2,343K: 1.17(6H,d,J=6.8Hz),1.80(4H,dd,J=5.5,5.7Hz),3.15(6H,s),3.21(4H,dd,J=5.5,5.7Hz),3.77(3H,s ),6.50(1H,dd,J=2.5,8.7Hz),6.62(1H,d,J=2.5Hz),7.25-7.28(1H,m),7.34(1H,d,J=8.7Hz),7.58(1H,br),7.77-7.79(1H,m),8.28(1H,s),8.49(1H,br),8.63(1H,br),9.25(1H,br)
ESI+: 543

### Step 6 Synthesis of 1-[3-methoxy-4-({4-[2-(propane-2-sulfonyl)anilino]-1,3,5-triazin-2-yl}amino)phenyl]piperidin-4-one (the compound of Formula (3))

To a solution comprising N-[4-(4,4-dimethoxypiperidin-1-yl)-2-methoxyphenyl]-N'-[2-(propane-2-sulfonyl)phenyl]-1,3,5-triazine-2,4-diamine (4.0 g), tetrahydrofuran (36 mL) and 2-propanol (4 mL), a mixture of 35% hydrochloric acid (1.44 g) and water (4 mL) was added, followed by stirring at room temperature for 17.5 hours. To the reaction mixture, a mixture of potassium carbonate (2.4 g) and water (4 mL) was added for extraction. An organic layer obtained was concentrated under reduced pressure. To a concentrated residue, acetone (12 mL) and water (4 mL) were added, and the resulting mixture was stirred for 30 minutes. After that, water (28 mL) was added thereto, the resulting mixture was stirred for 1 hour, and a precipitated crystal was collected by filtration. The obtained crystal was washed with a mixture of acetone (8 mL) and tetrahydrofuran (3 mL) and dried at 40°C under reduced pressure to give 1-[3-methoxy-4-({4-[2-(propane-2-sulfonyl)anilino]-1,3,5-triazin-2-yl}amino)phenyl]piperidin-4-one (3.42 g, 99.2% yield) as a crystal.
D2,343K: 1.17(6H,d,J=6.8Hz),2.46-2.50(4H,m),3.40(1H,sept,J=6.8Hz),3.61(4H,dd,J=6.1,6.2Hz),3.79(3H,s),6.57(1H,dd,J=2.6,8. 7Hz),6.70(1H,d,J=2.6Hz),7.25-7.29(1H,m),7.38(1H,d,J=8.7Hz),7.61(1H,br),7.77-7.80(1H,m),8.28(1H,s),8.50(1H,br),8.66(1H,br),9.25(1H,br)
ESI+: 497

### Step 7 Synthesis of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (the compound of Formula (1))

After 1-[3-methoxy-4-({4-[2-(propane-2-sulfonyl)anilino]-1,3,5-triazin-2-yl}amino)phenyl]piperidin-4-one (20.0 g), methylpiperazine (8.07 g), toluene (200 mL) and acetic acid (9.0 mL) were mixed, the mixture was stirred at room temperature for 1 hour. To the mixture, sodium triacetoxyborohydride (17.06 g) was added, followed by stirring at room temperature for 20 hours. The reaction mixture was extracted with water (60 mL) and methanol (20 mL) to give an organic layer and an aqueous layer 1. The organic layer was reextracted with water (20 mL) to give an aqueous layer 2. After mixing the aqueous layers 1 and 2, the resulting mixture was extracted with isopropyl acetate (200 mL). To an aqueous layer obtained, a mixture of methanol (220 mL), sodium hydroxide (9.68 g) and water (48 mL) was added, and the resulting mixture was seeded with a crystal (2.0 mg) of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine as a seed crystal. After stirring at room temperature for 1.5 hours, water (220 mL) was added thereto. The resulting mixture was further stirred at room temperature for 2.5 hours, and a precipitated crystal was collected by filtration. The obtained crystal was washed with a mixture of methanol (40 mL) and water (40 mL) and then dried at 50°C under reduced pressure to give N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (20.15 g, 86.1 % yield) as A06-type crystal.
D1: 1.31(6H,d,J=6.8Hz),1.59-1.78(2H,m),1.90-2.01(2H,m),2.24-2.80(11H,m),2.30(3H,s),3.19-3.32(1H,m),3.65-3.75(2H,m),3.88(3H,s),6.50-6.59(2H,m),7.18-7.30(1H,m),7.53-7.70(2H,m),7.88(1H,dd,J=1.5,8.3Hz),8.10(1H,br),8.37(1H,br),8.53(1H,br),9.29(1H,s) ESI+:581

### Alternative method 1 for Step 7 (an example using no seed crystal) Synthesis of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (the compound of Formula (1))

After 1-[3-methoxy-4-({4-[2-(propane-2-sulfonyl)anilino]-1,3,5-triazin-2-yl}amino)phenyl]piperidin-4-one (5.0 g), methylpiperazine (2.02 g), toluene (50 mL) and acetic acid (1.5 mL) were mixed, the mixture was stirred at room temperature for 1 hour. To the mixture, sodium triacetoxyborohydride (4.72 g) was added, followed by stirring at room temperature for 18 hours. The reaction mixture was extracted with water (15 mL) and methanol (5 mL) to give an organic layer and an aqueous layer 1. The organic layer was reextracted with water (5 mL) to give an aqueous layer 2. After mixing the aqueous layers 1 and 2, the resulting mixture was extracted with isopropyl acetate (50 mL). To an aqueous layer obtained, a mixture of methanol (55 mL), sodium hydroxide (2.0 g) and water (10 mL) was added. After stirring the resulting mixture at room temperature for 62 hours, water (55 mL) was added thereto and the resulting mixture was further stirred at room temperature for 2 hours. A precipitated crystal was collected by filtration. The obtained crystal was washed with a mixture of methanol (5 mL) and water (5 mL) and then dried at 40°C under reduced pressure to give N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (4.56 g, 78.0% yield) as A06-type crystal.
D1: 1.31(6H,d,J=6.8Hz),1.59-1.78(2H,m),1.90-2.01(2H,m),2.24-2.80(11H,m),2.30(3H,s),3.19-3.32(1H,m),3.65-3.75(2H,m),3.88(3H,s),6.50-6.59(2H,m),7.18-7.30(1H,m),7.53-7.70(2H,m),7.88(1H,dd,J=1.5,8.3Hz),8.10(1H,br),8.37(1H,br),8.53(1H,br),9.29(1+H,s)
ESI+: 581

### Alternative method 2 for Step 7 (an example using a reduction catalyst) Synthesis of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (the compound of Formula (1))

After 1-[3-methoxy-4-({4-[2-(propane-2-sulfonyl)anilino]-1,3,5-triazin-2-yl}amino)phenyl]piperidin-4-one (5.0 g), tetrahydrofuran (30 mL), methylpiperazine (1.81 g) and 10% palladium carbon (about 50% wet product, 0.8 g) were mixed, the mixture was stirred at 40°C for 7 hours under a hydrogen atmosphere (2.4821 × 10⁵ Pa). Palladium carbon was removed by filtration and washed with tetrahydrofuran (10 mL), and a filtrate obtained was concentrated under reduced pressure. To a concentrated residue, 2-butanone (9 mL) was added, and the resulting mixture was stirred at 60°C for 30 minutes and cooled slowly. After adding n-heptane (9 mL) at 30°C and stirring at room temperature for 19 hours, a precipitated crystal was collected by filtration. The obtained crystal was washed with a mixture of 2-butanone (1 mL) and n-heptane (1 mL) and then dried at 40°C under reduced pressure to give N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (3.09 g, 88.0% yield).
D1: 1.31(6H,d,J=6.8Hz),1.59-1.78(2H,m),1.90-2.01(2H,m),2.24-2.80(11H,m),2.30(3H,s),3.19-3.32(1H,m),3.65-3.75(2H,m),3.88(3H,s),6.50-6.59(2H,m),7.18-7.30(1H,m),7.53-7.70(2H,m),7.88(1H,dd,J=1.5,8.3Hz),8.10(1H,br),8.37(1H,br),8.53(1H,br),9.29(1H,s)
ESI+: 581

### Purification process using the tequnique of recrystallization of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (the compound of Formula (1))

(Method A): After N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (8.80 g) and 2-butanone (211 mL) were mixed, the mixture was stirred at 65°C for 30 minutes. After the dissolution was confirmed, clarification filtration was performed. The filtrate was concentrated at normal pressure to make a total volume of about 70 mL, cooled to 70°C, and then it was seeded with a crystal (0.9 mg) of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine as a seed crystal. The resulting mixture was stirred for about 10 minutes to give a slurry. After this slurry was stirred at 70°C for 3 hours, it was cooled to 5°C at a rate of 20°C/h and stirred for 17 hours, and then a precipitated crystal was collected by filtration. After the obtained crystal was washed with 2-butanone (35.2 mL) cooled with ice-cold water, the crystal was dried at 50°C under reduced pressure to give N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (7.88 g, 89.5% yield, 99.4% purity) as A04-type crystal (proportion of the A04-type crystal: 98.9%).

(Method B): After N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (8.80g), activated carbon (0.88 g) and 2-butanone (211 mL) were mixed, the mixture was stirred at 75°C for 1 hour, followed by filtration of activated carbon. To the filtrate, activated carbon (0.88 g) was added, and the resulting mixture was stirred at 75°C for 1 hour, followed by filtration of activated carbon. To the filtrate, activated carbon (0.88 g) was added, and the resulting mixture was stirred at 75°C for 1 hour, followed by filtration of activated carbon. The filtrate was concentrated at normal pressure to make a total volume of about 70 mL, cooled to 70°C, and then it was seeded with a crystal (0.9 mg) of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine as a seed crystal. The resulting mixture was stirred for about 10 minutes to give a slurry. After this slurry was stirred at 70°C for 3 hours, it was cooled to 5°C at a rate of 20°C/h and stirred for 16 hours, and then a precipitated crystal was collected by filtration. After the obtained crystal was washed with 2-butanone (35.2 mL) cooled with ice-cold water, the crystal was dried at 50°C under reduced pressure to give N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (6.60 g, 75.0% yield, 99.3% purity) as A04-type crystal (proportion of the A04-type crystal: 100%)

### Example 2

### Step 1 Synthesis of 4,4-dimethoxy-1-(3-methoxy-4-nitrophenyl)piperidine (the compound of Formula (10) wherein R¹ and R² are both methyl)

After 4,4-dimethoxypiperidine monohydrochloride (69.9 kg) and N,N-dimethylformamide (125.7 kg) were mixed, 1,8-diazabicyclo[5.4.0]undec-7-ene (117.3 kg) and N,N-dimethylformamide (17.0 kg) were added to the mixture. To the resulting mixture, a N,N-dimethylformamide (57.0 kg) solution of 5-fluoro-2-nitroanisole (60.0 kg) which was separately prepared was added at room temperature, and a N,N-dimethylformamide (29.0 kg) solution was added thereto, followed by stirring for 5 hours. To the reaction mixture, 4,4-dimethoxy-1-(3-methoxy-4-nitrophenyl)piperidine (about 6 g) was added as a seed crystal at room temperature, and the resulting mixture was stirred at room temperature for 14 hours. To the reaction mixture, water (240 kg) was added at room temperature, and the resulting mixture was stirred for 22 hours, and then a precipitated crystal was collected by filtration. The obtained crystal was washed with a mixture of N,N-dimethylformamide (56.9 kg) and water (60 kg) and then washed twice with water (120 kg) and dried at 50°C under reduced pressure to give 4,4-dimethoxy-1-(3-methoxy-4-nitrophenyl)piperidine (99.7 kg, 96.0% yield) as a crystal.

D2: 1.72-1.80(4H,m),3.14(6H,s),3.44-3.50(4H,m),3.91(3H,m),6.52(1H,d,J=2.4Hz),6.60(1H,dd,J=2.4,9.2Hz),7.88(1H,d,J=9.2Hz) ESI+: 297

### Step 2 Synthesis of 4-(4,4-dimethoxypiperidin-1-yl)-2-methoxyaniline (the compound of Formula (6) wherein R¹ and R² are both methyl)

After 4,4-dimethoxy-1-(3-methoxy-4-nitrophenyl)piperidine (99.0 kg), 5% palladium carbon (about 50% wet product, 10.5 kg) and tetrahydrofuran (440 kg) were mixed at room temperature, the mixture was stirred at room temperature for 3 hours under a hydrogen atmosphere (200-300 kPa). After that, palladium carbon was removed by filtration and washed with tetrahydrofuran (180.5 kg). Subsequently, the filtrate was concentrated under reduced pressure to make a total volume of about 220 L and seeded with a crystal (about 10 g) of 4-(4,4-dimethoxypiperidin-1-yl)-2-methoxyaniline as a seed crystal. To a slurry obtained, n-heptane (205.4 kg) was added at 40°C, and the resulting mixture was stirred for 1 hour and then cooled to 0°C and stirred for 16 hours. To the slurry, n-heptane (613.5 kg) was added and the resulting mixture was stirred for 2 hours, and then a crystal was collected by filtration. The obtained crystal was washed with a mixture of tetrahydrofuran (17.8 kg) and n-heptane (81.5 kg) and dried at 50°C under reduced pressure to give 4-(4,4-dimethoxypiperidin-1-yl)-2-methoxyaniline (84.1 kg; 94.5% yield) as a crystal.

D2: 1.72-1.80(4H,m),2.90-2.97(4H,m),3.11(6H,s),3.73(3H,m),4.21(1H,br),6.30(1H,d,J=2.4,8.4Hz),6.46_6.56(2H,m) ESI+: 267

### Step 3 Synthesis of 4,6-dichloro-N-[2-(propane-2-sulfonyl)phenyl]-1,3,5-triazin-2-amine (the compound of Formula (7) wherein Lv are both Cl)

Cyanuric chloride (40.0 kg) and acetone (249.2 kg) were mixed at 17°C. To the mixture, sodium hydrogen carbonate (21.9 kg) and 2-(isopropylsulfonyl)aniline (47.5 kg) were added, followed by stirring at room temperature for 23 hours. To the reaction mixture, water (320 kg) was added at room temperature, and then the resulting mixture was stirred for 3.5 hours and a precipitated crystal was collected by filtration. The obtained crystal was washed with a mixture of acetone (63.0 kg) and water (80 kg) and then dried at 50°C under reduced pressure to give 4,6-dichloro-N-[2-(propane-2-sulfonyl)phenyl]-1,3,5-triazin-2-amine (71.6 kg; 95.1 % yield) as a crystal.

D1: 1.32(6H,d,J=6.8Hz),3.22(1H,sept,J=6.8Hz),7.37(1H,m),7.74(1H,m),7.93(1H,m),8.44(1H,m), 10.02(1H,br)
ESI-: 345,347

### Step 4 Synthesis of 6-chloro-N-[4-(4,4-dimethoxypiperidin-1-yl)-2-methoxyphenyl]-N'-[2-(propane-2-sulfonyl)phenyl]-1,3,5-triazine-2,4-diamine (the compound of Formula (5) wherein Lv is Cl and R¹ and R² are both methyl)

After 4,6-dichloro-N-[2-(propane-2-sulfonyl)phenyl]-1,3,5-triazin-2-amine (70.9 kg) and tetrahydrofuran (611.1 kg) were mixed at room temperature, 4-(4,4-dimethoxypiperidin-1-yl)-2-methoxyaniline (57.1 kg) and N,N-diisopropylethylamine (29.1 kg) were added to the mixture at room temperature. The resulting mixture was stirred for 4 hours. After that, isopropyl acetate (61.0 kg) and then a mixture of potassium carbonate (3.6 kg) and water (71 kg) were added thereto for extraction. An organic layer obtained was concentrated under reduced pressure at an outside temperature of about 40°C to make a total volume of about 360 L and seeded with a crystal (about 7 g) of 6-chloro-N-[4-(4,4-dimethoxypiperidin-1-yl)-2-methoxyphenyl]-N'-[2-(propane-2-sulfonyl)phenyl]-1,3,5-triazine-2,4-diamine as a seed crystal to give a slurry. To this slurry, 2-propanol (111.0 kg) and n-heptane (243.1 kg) were added, and the resulting mixture was cooled to room temperature for 2 hours. Subsequently, the mixture was cooled to 0°C and stirred for 18 hours, and a precipitated crystal was collected by filtration. The obtained crystal was washed with a mixture of tetrahydrofuran (74.9 kg), 2-propanol (44.6 kg) and n-heptane (97.6 kg) and then dried at 50°C under reduced pressure to give 6-chloro-N-[4-(4,4-dimethoxypiperidin-1-yl)-2-methoxyphenyl]-N'-[2-(propane-2-sulfonyl)phenyl]-1,3,5-triazine-2,4-diamine (108.9 kg, 92.4% yield) as a crystal.

D1: 1.30(6H,d,J=6.8Hz),1.88-1.92(4H,m),3.18-3.26(1H,m),3.23(3H,s),3.87(1H,br),6.53(2H,br),7.21-7.23(1H,m),7.62(1H,br),7.88(1H,d,J=7.9Hz),8.05(1H,br),8.48(1H,br),9.41(1H,br)
ESI-: 575,577

### Steps 5 and 6 (successive steps) Synthesis of 1-[3-methoxy-4-({4-[2-(propane-2-sulfonyl)anilino]-1,3,5-triazin-2-yl}amino)phenyl]piperidin-4-one) (the compound of Formula (3))

After 6-chloro-N-[4-(4,4-dimethoxypiperidin-1-yl)-2-methoxyphenyl]-N'-[2-(propane-2-sulfonyl)phenyl]-1,3,5-triazine-2,4-diamine (108.2 kg), tetrahydrofuran (866.0 kg) and 10% palladium carbon (about 50% wet product, 23.3 kg) were mixed, N,N-diisopropylethylamine (28.9 kg) and 2-propanol (85.5 kg) were added to the mixture. The resulting mixture was stirred at 40°C for 4 hours under a hydrogen atmosphere (100-300 kPa). Palladium carbon was removed by filtration and washed with tetrahydrofuran (193.3 kg) to give a solution comprising N-[4-(4,4-dimethoxypiperidin-1-yl)-2-methoxyphenyl]-N'-[2-(propane-2-sulfonyl)phenyl]-1,3,5-triazine-2,4-diamine. To this solution, a mixture of 35% hydrochloric acid (39.1 kg) and water (217 kg) was added, and the resulting mixture was stirred at room temperature for 15.5 hours. To the reaction mixture, a mixture of potassium carbonate (64.8 kg) and water (217 kg) was added for extraction. To an organic layer obtained, activated carbon (10.8 kg) was added, and the resulting mixture was stirred at room temperature for 17 hours. After that, the mixture was filtered and activated carbon was washed with tetrahydrofuran (96.0 kg). The filtrate obtained was concentrated at 40°C under reduced pressure to make a total volume of about 380 L. Acetone (257.1 kg) was added to the resulting mixture, followed by seeding with a crystal (about 11 g) of 1-[3-methoxy-4-({4-[2-(propane-2-sulfonyl)anilino]-1,3,5-triazin-2-yl}amino)phenyl]piperidin-4-one as a seed crystal. The resulting mixture was stirred for 1 hour and then water (865 kg) was added thereto, followed by stirring for 15 hours. A precipitated crystal was collected by filtration. The obtained crystal was washed with a mixture of acetone (50.9 kg) and water (173 kg) and dried at 50°C under reduced pressure to give 1-[3-methoxy-4-({4-[2-(propane-2-sulfonyl)anilino]-1,3,5-triazin-2-yl}amino)phenyl]piperidin-4-one (82.9 kg, 89.0% yield (yield of the successive two steps)) as a crystal.

D2,343K: 1.17(6H,d,J=6.8Hz),2.46-2.50(4H,m),3.40(1H,sept,J=6.8Hz),3.61(4H,dd,J=6.1,6.2Hz),3.79(3H,s),6.57(1H,dd,J=2.6,8. 7Hz),6.70(1H,d,J=2.6Hz),7.25-7.29(1H,m),7.38(1H,d,J=8.7Hz),7.61(1H,br),7.77-7.80(1H,m),8.28(1H,s),8.50(1H,br),8.66(1H,br),9.25(1H,br)
ESI+: 497

### Step 7 Synthesis of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (the compound of Formula (1))

After 1-[3-methoxy-4-({4-[2-(propane-2-sulfonyl)anilino]-1,3,5-triazin-2-yl}amino)phenyl]piperidin-4-one (60.1 kg), methylpiperazine (24.2 kg), toluene (500 kg) and acetic acid (28.4 kg) were mixed, the mixture was stirred at room temperature for 1 hour. To this mixture, sodium triacetoxyborohydride (51.4 kg) was added, followed by stirring at room temperature for 17 hours. The reaction mixture was extracted with methanol (47.5 kg) and water (180.1 kg) to give an organic layer and an aqueous layer 1. The oraganic layer was reextracted with water (60.0 kg) to give an aqueous layer 2. After mixing the aqueous layers 1 and 2, the resulting mixture was extracted with isopropyl acetate (523.4 kg). To an aqueous layer obtained, a mixture of methanol (522.3 kg), 48% sodium hydroxide (60.6 kg) and water (112.7 kg) was added, and the resulting mixture was seeded with a crystal (about 6 g) of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine as a seed crystal. After stirring at room temperature for 2 hours, water (660.2 kg) was added thereto. The resulting mixture was further stirred at room temperature for 3.5 hours, and a precipitated crystal was collected by filtration. The obtained crystal was washed with a mixture of methanol (104.4 kg) and water (132.0 kg) and then dried at 50°C under reduced pressure to give N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (54.2 kg, 77.1% yield) as A06-type crystal.

D1: 1.31(6H,d,J=6.8Hz),1.59-1.78(2H,m),1.90-2.01(2H,m),2.24-2.80(11H,m),2.30(3H,s),3.19-3.32(1H,m),3.65-3.75(2H,m),3.88(3H,s),6.50-6.59(2H,m),7.18-7.30(1H,m),7.53-7.70(2H,m),7.88(1H,dd,J=1.5,8.3Hz),8.10(1H,br),8.37(1H,br),8.53(1H,br),9.29(1H,s)
ESI+: 581

### Purification process using the technique of recrystallization of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (the compound of Formula (1))

After N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (54.3 kg), activated carbon (5.4 kg) and 2-butanone (1046.1 kg) were mixed, the mixture was stirred at 75°C for 1 hour, followed by filtration of activated carbon. To the filtrate, activated carbon (5.4 kg) was added, and the resulting mixture was stirred at 75°C for 1 hour, followed by filtration of activated carbon. To the filtrate, activated carbon (5.4 kg) was added, and the resulting mixture was stirred at 75°C for 1 hour, followed by filtration of activated carbon. The filtrate was concentrated at normal pressure to make a total volume of about 430 L, cooled to 70°C, and then seeded with a crystal (about 5 g) of N-{2-methoxy-4-[4-(4-methylpiperazin-1-yl)piperidin-1-yl]phenyl}-N'-[2-(propane-2-sulfonyl)phenyl]-1,3,5-triazine-2,4-diamine as a seed crystal. The resulting mixture was stirred for 3 hours and then cooled to 5°C at a rate of 20°C/h, and a precipitated crystal was collected by filtration. After the obtained crystal was washed with 2-butanone (220 L) cooled to 5°C, the crystal was dried at 50°C under reduced pressure to give N-{2-methoxy-4-[4-(4-methylpiperazin-1-yl)piperidin-1-yl]phenyl}-N'-[2-(propane-2-sulfonyl)phenyl]-1,3,5-triazine-2,4-diamine (42.6 kg, 78.5% yield, 99.5% purity) as A04-type crystal (proportion of the A04-type crystal: 100%).

The effects of the present invention are described below.
The yields of the production methods in Examples 1 and 2 according to the present invention are respectively shown in Tables 1 and 2 below. It is to be noted that since a plurality of steps are described for Steps 4 to 7, Tables 1 and 2 indicate the yields of the steps described first among those plural steps.

**[Table 1]**

| Example 1 | Yield |
|---|---|
| Step 1 | 96.1% |
| Step 2 | 93.7% |
| Steps 1-2 (Total) | 90.0% |
| Step 3 | 95.8% |
| Step 4 | 92.4% |
| Steps 5-6 | 88.7% |
| Step 7 | 86.1% |
| Steps 3-7 (Total) | 67.6% |
| Total for all steps | 60.8% |

**[Table 2]**

| Example 2 | Yield |
|---|---|
| Step 1 | 96.0% |
| Step 2 | 94.5% |
| Steps 1-2 (Total) | 90.7% |
| Step 3 | 95.1% |
| Step 4 | 92.4% |
| Steps 5-6 | 89.0% |
| Step 7 | 77.1% |
| Steps 3-7 (Total) | 60.3% |
| Total for all steps | 54.7% |

Meanwhile, the yields of the known method for producing the compound of Formula (1) in the Reference Example are as shown in Table 3 below.

**[Table 3]**

| | Yield |
|---|---|
| Step 1 | 78.9% |
| Step 2 | 73.2% |
| Step 3 | 98.8% |
| Steps 1-3 (Total) | 57.1% |
| Step 4^{*1} | 55.9% |
| Step 4 (Alternative method)^{*2} | 36.1% |
| Step 5 | 50.1% |
| Steps 4-5 (Total) | 18.1^{*3}, 28.0% |
| Total for all steps | 10.3^{*3}, 16.0% |

| | |
|---|---|
| ^{*1}: The calculation is based on the compound of Formula (8), since the reaction in Step 4 of the Reference Example was performed using about 1.5 equivalents of the compound of Formula (15) relative to the compound of Formula (8). ^{*2} : In Step 4 (alternative method) of the Reference Example, the reaction was performed using almost equal amounts of the compounds of Formulae (15) and (8). The yields of the production method of the present invention in the Example shown in Table 1 were calculated based on cyanuric chloride (one embodiment of the compound of Formula (9)) which is a starting material. To compare yields based on corresponding starting materials, the yield of Step 4 (alternative method) of the Reference Example was calculated based on the compound of Formula (15) (2,4-dichloro-1,3,5-triazine). It is to be noted that the reaction shown in Preparation Example 7 of Patent Document 1 (a preparation method corresponding to Step 4 of the Reference Example) was performed using almost equal amounts of 2,4-dichloro-6-methoxy-1,3,5-triazine and 2-(propane-2-sulfonyl)aniline (yield: 28.3%). ^{*3}: Total yields or overall yields obtained when the alternative method for Step 4 was employed. | |

Tables 1 to 3 shown above confirmed that the production method of the present invention (Table 1 or 2) had good yields as compared with the known production method (Table 3). Further, the production method of the present invention did not include a step with a yield of 60% or below, could maintain a high overall yield for all the steps, and was also advantageous in terms of cost. Moreover, since the yields from the kilogram-scale production (Table 2) are almost the same as those from the gram-scale production (Table 1), the production method of the present invention is suitable for industrial production. Further, the production method of the present invention in no way requires purification using silica gel column chromatography, nor is there any concern about the formation of a mutagenic mesylate ester as a by-product. These findings confirmed that the production method of the present invention is a method for producing the compound of Formula (1) that is superior and suitable especially for the industrial production of pharmaceutical products.

In the production method of the Reference Example, particularly the yields of Steps 4 and 5 are so low as to be below 60%. In contrast, the yields of Steps 3 and 4 in the Examples of the present invention are all so high as to be above 90%, which shows great improvement in yields. This improvement in yields was finally achieved as a result of using predetermined reactive starting materials and synthetic intermediates (in particular, the compounds of Formulae (9) and (7)) and predetermined synthetic intermediates (in particular, the compound of Formula (6)) which are suitable for the reactions with the starting materials and synthetic intermediates as well as going through numerous trials and errors concerning reaction conditions (in particular, basicity, reaction temperatures, solvents).

It is to be noted that it is easy to conceive of reacting the compound of Formula (13) shown in Reaction Scheme (I), instead of the compound of Formula (6), with the compound of Formula (7), in Step 4 of Reaction Scheme (II). However, it was found that when cyanuric chloride is used as the compound of Formula (9), the compound of Formula (21) synthesized by the subsequent step for reacting the compounds of Formulae (7) and (13) is extremely unstable and that a large volume of the compound of Formula (22) is produced as a by-product, in addition to the compound of Formula (21).

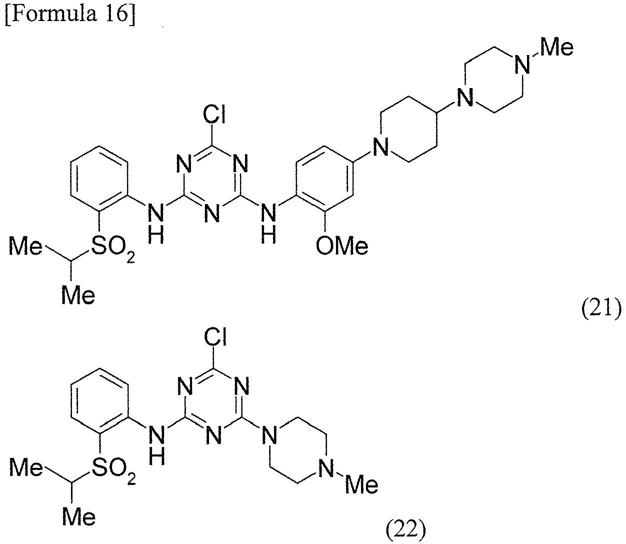

Hence, in order to achieve high-yield production of the compound of Formula (1), the compound of Formula (1) is suitably produced through a process in which the compound of Formula (9) is used as a starting material to give the compound of Formula (7), which is subsequently reacted with the compound of Formula (6), instead of the compound of Formula (13), as shown in Reaction Scheme (II) as the production method of the present invention.

Further, cyanuric chloride, which is used as one embodiment of the compound of Formula (9) in the production method of the present invention in the Examples, can be produced on a large scale and is also mass-produced as a general chemical product. Meanwhile, the compound of Formula (15) has problems about stable procurement. The method for producing the compound, which is shown in Reaction Scheme (III) (refer to Synthesis, 11,907, 1981; 69% yield), uses a synthetic intermediate N-cyano-chloroform amidine, which is thermally unstable and thus degraded in a drying process and causes rapid exotherm due to the degradation (at the level slightly below the hazard assessment line for Category 5 (self-reactive substances) of hazardous materials according to the Japanese Fire Service Act). Hence, the compound of Formula (15) is feared to have risks including explosion and the like in large-scale production. Further, the reaction conditions are limited in the method for producing the compound in that the number of the groups replaced by Cl on the triazine is limited to 2; partially for this reason, no method for producing the compound efficiently has been reported so far except for the method shown by Reaction Scheme (III). In light of these matters, it has to be admitted that the compound of Formula (15) is unsuitable as a starting material for industrial production in terms of large-scale and stable procurement.

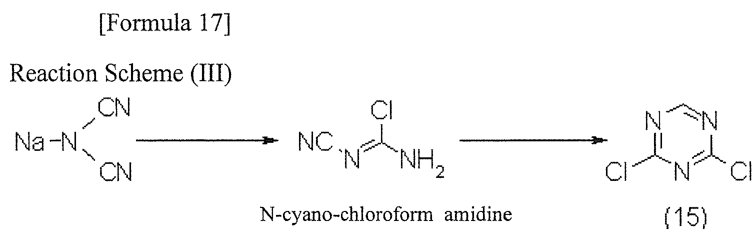

These matters indicate that the production method of the present invention in which cyanuric chloride, which is one embodiment of the compound of Formula (9), is used as a starting material, instead of the compound of Formula (15), is superior in terms of stable procurement of starting material and cost.

Further, the production yield of 4-(4,4-dimethoxypiperidin-1-yl)-2-methoxyaniline (the compound of Formula (6') wherein Rb¹ and Rb² are both methyl) (the total yield of Steps 1 to 2 in Table 1) is very high and suitable for industrial production, as compared with the production yield of the compound of Formula (13) (the total yield of Steps 1 to 3 in Table 3), and the compound of Formula (6') is a synthetic intermediate that is also superior in terms of cost.

It is clear from these matters how the method of the present invention is industrially superior to known methods. In particular, the production method of the present invention can achieve high overall yields and this is believed to have led to the establishment of the production method that can be industrially used.

### INDUSTRIAL APPLICABILITY

The present invention provides a method for producing the compound of Formula (1) which is high yield and low cost and suitable for the industrial production of pharmaceutical products, and also provides a synthetic intermediate that is useful in the production method.

## Claims

1. A method for producing the compound of Formula (1): 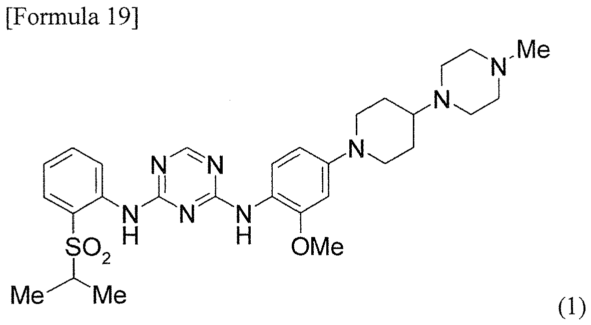 comprising subjecting the compound of Formula (3): 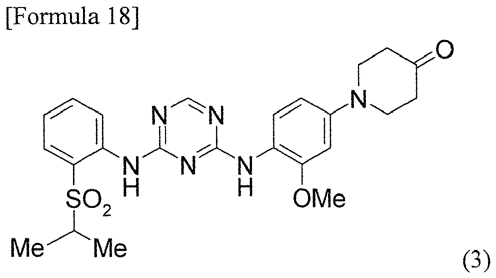 to a reductive amination reaction by allowing 1-methylpiperazine to act on the compound of Formula (3).

2. The method according to Claim 1, wherein the compound of Formula (3) is produced by a method comprising subjecting a compound of Formula (4): 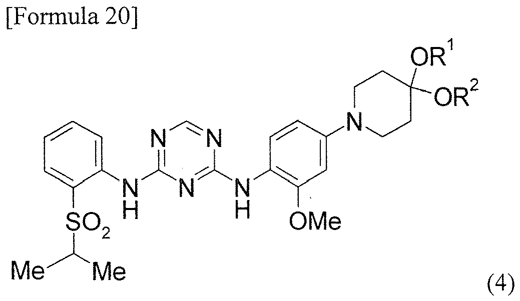 (wherein R¹ and R², which may be the same or different, are lower alkyl, or R¹ and R² taken together may form lower alkylene)
to a deketalization reaction.

3. The method according to Claim 2, wherein the compound of Formula (4) is produced by a method comprising subjecting a compound of Formula (5): 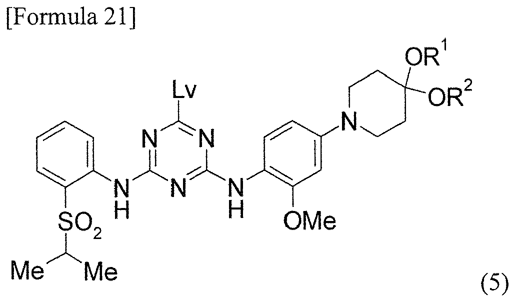 (wherein R¹ and R², which may be the same or different, are lower alkyl, or R¹ and R² taken together may form lower alkylene; Lv is a leaving group)
to a hydrogenation reaction.

4. The method according to Claim 3, wherein the compound of Formula (5) is produced by a method comprising subjecting a compound of Formula (7): 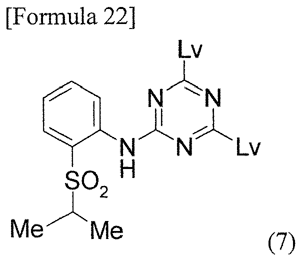 (wherein Lv, which may be the same or different, are leaving groups)
to an aromatic nucleophilic substitution reaction by allowing a compound of Formula (6): 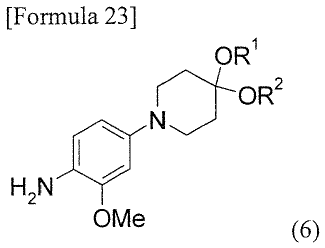 (wherein R¹ and R², which may be the same or different, are lower alkyl, or R¹ and R² taken together may form lower alkylene)
to act on the compound of Formula (7).

5. The method according to Claim 4, wherein the compound of Formula (7) is produced by a method comprising subjecting a compound of Formula (9): 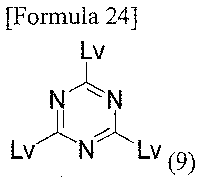 (wherein Lv, which may be the same or different, are leaving groups)
to an aromatic nucleophilic substitution reaction by allowing 2-(isopropylsulfonyl)aniline to act on the compound of Formula (9).

6. The method according to Claim 4 or 5, wherein the compound of Formula (6) is produced by a method comprising:
subjecting a compound of Formula (12): 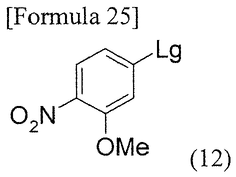 (wherein Lg is a leaving group)
to an aromatic nucleophilic substitution reaction by allowing a compound of Formula (11): 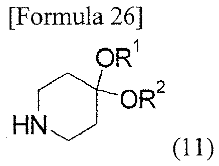 (wherein R¹ and R², which may be the same or different, are lower alkyl, or R¹ and R² taken together may form lower alkylene)
to act on the compound of Formula (12); and
then performing a hydrogenation reaction.

7. A compound represented by Formula (4') or a salt thereof: 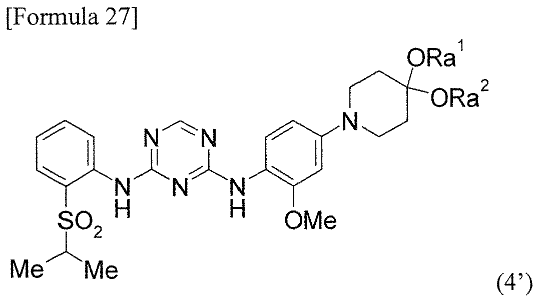 (wherein Ra¹ and Ra², which may be the same or different, are lower alkyl, or Ra¹ and Ra² taken together may form lower alkylene, provided that Ra¹ and Ra² taken together do not form dimethylene).

8. A compound represented by Formula (5) or a salt thereof: 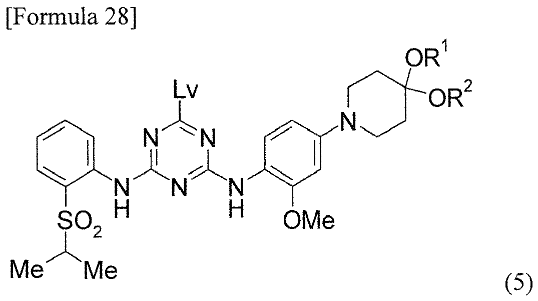 (wherein R¹ and R², which may be the same or different, are lower alkyl, or R¹ and R² taken together may form lower alkylene; Lv is a leaving group).

9. The compound according to Claim 8 or a salt thereof, wherein Lv is a leaving group selected from the group consisting of halogen, sulfonyloxy groups, lower alkylsulfanyl and lower alkylsulfonyl.

10. The compound according to Claim 9, wherein Lv is halogen.

11. The compound according to Claim 10, wherein Lv is Cl and R¹ and R² are both methyl groups.

12. The compound according to Claim 7 or a salt thereof, wherein Ra¹ and Ra² are both methyl groups.

13. A compound represented by Formula (6') or a salt thereof: 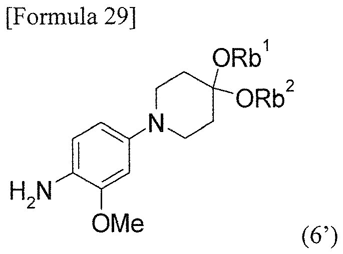 (wherein Rb¹ and Rb², which may be the same or different, are lower alkyl, or Rb¹ and Rb² taken together may form lower alkylene, provided that Rb¹ and Rb² taken together do not form dimethylene).

14. The compound according to Claim 13, wherein Rb¹ and Rb² are both methyl groups.

15. The method according to Claim 5, wherein the compound of Formula (9) is cyanuric chloride.

16. The method according to any of Claims 1 to 6, further comprising a purification step including crystallization of the compound of Formula (1).
